(19) Europäisches Patentamt

European Patent Office

Office européen des brevets

(11) **EP 3 699 271 B1**

(12) **EUROPEAN PATENT SPECIFICATION**

(45) Date of publication and mention
of the grant of the patent:
**25.09.2024   Bulletin 2024/39**

(21) Application number: **20152501.1**

(22) Date of filing: **01.02.2013**

(51) International Patent Classification (IPC):
*C12N 9/16* $^{(2006.01)}$          *C12N 9/96* $^{(2006.01)}$
*A23K 10/14* $^{(2016.01)}$        *A23K 20/10* $^{(2016.01)}$
*A23K 20/189* $^{(2016.01)}$      *A23K 20/26* $^{(2016.01)}$
*A23K 40/10* $^{(2016.01)}$        *A23K 40/20* $^{(2016.01)}$
*A23K 40/30* $^{(2016.01)}$        *A23L 29/00* $^{(2016.01)}$
*A23K 40/25* $^{(2016.01)}$

(52) Cooperative Patent Classification (CPC):
**A23K 40/25; A23K 10/14; A23K 20/10;
A23K 20/189; A23K 20/26; A23K 40/10;
A23K 40/20; A23K 40/30; A23L 29/05;
A23L 29/06; C12N 9/16; C12N 9/96;
C12Y 301/03008;** C12Y 301/03

(54) **GRANULE AND PELLET COMPOSITIONS COMPRISING PHYTASE AND PHYTIC ACID AND PREPARATION METHOD**

GRANULARE UND PELLETZUSAMMENSETZUNGEN MIT PHYTASE UND PHYTINSÄURE UND HERSTELLUNGSVERFAHREN

COMPOSITIONS GRANULAIRES ET PELLETS COMPRENANT DE LA PHYTASE ET DE L'ACIDE PHYTIQUE ET PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **07.02.2012   US 201261595923 P**

(43) Date of publication of application:
**26.08.2020   Bulletin 2020/35**

(62) Document number(s) of the earlier application(s) in
accordance with Art. 76 EPC:
**13705655.2 / 2 811 844**

(73) Proprietor: **Danisco US Inc.**
**Palo Alto, CA 94304 (US)**

(72) Inventor: **Mark, GEBERT**
**Palo Alto, CA California 94304 (US)**

(74) Representative: **Mewburn Ellis LLP**
**Aurora Building**
**Counterslip**
**Bristol BS1 6BX (GB)**

(56) References cited:
**EP-A1- 0 969 089          WO-A1-93/16175**
**WO-A2-03/037102        WO-A2-2004/071218**
**WO-A2-2006/043178      US-A1- 2010 004 170**

- **CASEY A AND WALSH G: "The stabilising effect of the substrate phytic acid on the enzyme phytase, in animal feed", BIOCHEMICAL SOCIETY TRANSACTIONS, vol. 28, no. 1, 73, 2000, pages A42, XP008164363**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**TECHNICAL** FIELD

The present invention relates to a granule composition comprising phytic acid and phytase, a pellet composition comprising this granule composition and a method for producing a heat-treated food or animal feed pellet.

**BACKGROUND**

The use of active agents, such as enzymes, in foods and animal feed is common. Enzymes are known to improve digestibility of food or animal feed, reduce anti-nutritional factors in food and animal feed, and improve animal productivity.

When compared with dry feed mixes, feed pellets have properties that are favored by the industry, such as improved feed quality, decreased pathogens, lower dust levels during manufacture, good handling, and more uniform ingredient dosing.

Inactivation of enzymes can occur during industrial food and feed processes (such as pelleting) by, for example, heat treatment, high pressure, shear stress, and chemical treatment (such as pH, surfactant, and solvents). The inactivation is at least partially reversible if the enzyme reactivates after processing, for example, upon cooling after steam treatment and pelleting; the inactivation is irreversible if the catalytic activity does not resume after processing, for example, upon cooling after steam treatment and pelleting. The irreversible inactivation and reduced activity of an enzyme is generally not desirable in processes such as pelleting.

Preferred industry pelleting processes utilize steam injection, in a process known as conditioning, which adds moisture and elevates the temperature prior to the pelleting step which forces the steam heated feed ingredients, or conditioned mash, through a die. The pelleting process temperatures may be from about 70C to 95C, or higher.

Because of the steam, temperatures, compression forces and chemicals used in pelleting processes, the activity or potency of enzymes are often significantly reduced during processing and subsequent storage. This problem is illustrated by the fact that feed enzymes are often provided to the industry as stabilized liquid products that are sprayed onto feed pellets after the pelleting process to avoid enzyme inactivation. Homogeneous dosing is difficult to achieve when the enzyme is applied post pelleting, for instance, by spraying the enzyme onto the pellets, and the cost of the equipment to add enzyme post-pelleting is high. Alternatively, liquid enzyme formulations, or dry mix enzyme formulations, may be added to the mixer prior to pelleting. In certain instances, higher levels of enzymes than otherwise needed may be added in order to compensate for losses during pelleting.

There is a need in the food and feed industries for stable, durable enzyme granules to serve as components in formulations that are subjected to steam treatment pelleting processes without appreciable loss of enzyme activity.

Approaches to avoid the problem of irreversibly inactivating enzymes or reducing the activity of the enzyme in industrial processes include identifying new sources of an enzyme (e.g. the identification of a known enzyme in an extreme thermophile microorganism) or identifying means to stabilize known enzymes.

Klibanov, 1983, (Stabilization of Enzymes against Thermal Inactivation, Advances in Applied Microbiology, volume 29, page 1-28) discloses that there are three basic means for stabilizing enzymes: (1) immobilization, (2) chemical modification and (3) inclusion of additives. However, Klibanov (1983) further discloses that any one of these methods could lead to stabilization or destabilization, or have no effect at all.

Pace and Grath (J Biol Chem 255:3862, 1980) state that an increased stability of an enzyme in the presence of its substrate, coenzyme, or any small molecule that it binds specifically results because binding to the native state shifts the unfolding equilibrium and decreases the concentration of unfolded states of the enzyme.

EP0969089 discloses that amino acid substitutions may affect the stability of phytase with some having a stabilizing effect and others having a destabilizing effect, and some amino acid substitutions having no effect at all.

Phytic acid (or phytate when in the salt form) is found in many plant tissues especially bran, seeds and cereals. Phytate phosphorus is poorly digested by monogastric animals such as swine, poultry, fish and man because they lack, or have low levels of, the digestive enzyme phytase in the intestine.

EP0619369 and US5554399 disclose enzyme compositions comprising a phytase and an acid phosphatase and use of the enzyme composition in food, pelleted feed and fodder.

WO2004071218 discloses increasing the amount of minerals in a food. WO2004071218 discloses a preparation comprising an active phytase, a phytate and an essential cation. WO2004071218 discloses that the preparation may be added to any food or drink product for human consumption or to condiments such as curry powder.

WO03/037102 discloses methods for improving the nutritional value of foodstuffs containing myo-inositol hexakisphosphate by feeding the foodstuff in combination with a phytase expressed in yeast.

EP0420428 discloses a method for treating rheumatoid arthritis by the use of a mixture of phytic acid, a phytate salt, or an isomer or hydrolysate of phytic acid (including inositol) or a phytate salt in combination with a dephosphorylating enzyme. The phytic acid or phytate salt may be adsorbed onto a solid carrier for administration. For oral administration, the composition further comprises a dephosphorylating enzyme such as a phytase.

US4952396 discloses a method for inhibiting tumor growth by administering a compound selected from the group consisting of phytic acid, a phytate salt, an isomer or hydrolysate of phytic acid (including inositol) or a phytate salt, or a mixture of any combination of these. The phytic acid or phytate salt may be adsorbed onto a solid carrier for administration. For oral administration, the composition further comprises a dephosphorylating enzyme such as a phytase.

US5112814 discloses a method for treating Parkinson's disease by administering a compound selected from the group consisting of phytic acid, a phytate salt, an isomer or hydrolysate of phytic acid (including inositol) or a phytate salt, or a mixture of any combination of these. The phytic acid or phytate salt may be adsorbed onto a solid carrier for administration. For oral administration, the composition may be co-administered with an enzyme which hydrolyses phosphate groups such as a phytase.

US4758430 discloses a method for treating Alzheimer's disease by administering a compound selected from the group consisting of phytic acid, a phytate salt, an isomer or hydrolysate of phytic acid or a phytate salt, or a mixture of any combination of these. The phytic acid or phytate salt may be adsorbed onto a solid carrier for administration. For oral administration, the composition further comprises a dephosphorylating enzyme such as a phytase.

EP0969089 discloses a stabilizing enzyme formulation comprising phytase and one or more stabilizing agents selected from the group consisting of: $C_5$ sugars; polyethylene glycols having a molecular weight of 600 to 4000 Da; disodium salts of malonic, glutaric and succinic acid; carboxymethylcellulose; and alginate. As an alternative, EP0969089 discloses that phytase can be stabilized by crosslinking with glutaldehyde or by oxidation with sodium periodate and reaction with adipic acid dihydrazine.

US 2010/0004170 describes increasing the stability of protein-containing materials by applying a coating layer comprising a micronized product from leguminous plants.

WO9316175 discloses a liquid phytase formulation stabilized by the addition of urea and/or a polyol such as sorbitol and glycerol, and applied to pelleted feed matter. The stabilized liquid formulations of WO9316175 are more resistant to heat inactivation of enzyme activity and may be stored for longer periods of time with better retention of phytase activity.

Singh and Satyanarayana (Bioresource Technology, 2009; 100: 2046-2051) disclose the purification and characterization of a histidine acid phosphatase (HAP) phytase from the thermophilic mould *Sporotrichum thermophile.* Singh and Satyanarayana (2009) determined the thermostability of the phytase by incubating the enzyme at 60°C and 80°C at pH 3.0, 5.0 and 7.0 buffers. Singh and Satyanarayana (2009) determined the effect of the stabilizers glycerol, trehalose, sorbitol and phytic acid on enzyme activity at 80°C.

WO 2006/043178 describes a phytase polypeptide obtained from *Buttiauxella* sp., e.g. *Buttiauxella* sp. strain P1-29.

The present teachings seek to overcome some of these problems. For example, the present teachings can address problems associated with the effect of food and animal feed industrial processes on phytase.


**SUMMARY**

The scope of protection is strictly defined by the appended set of claims.

The invention provides a granule composition comprising phytic acid and phytase, wherein the phytase and the phytic acid are in adjacent coating layers of a multi-layered granule, or phytase is in the core of the granule and phytic acid is in the adjacent coating layer, wherein the phytic acid is at least 10 millimolal in said composition, and wherein the phytic acid is selected from inositol hexaphosphate, inositol pentaphosphate, inositol tetraphosphate, inositol triphosphate, inositol diphosphate and inositol monophosphate, or salts or mixtures thereof.

The phytic acid may be at least 20 millimolal, at least 30 millimolal, at least 50 millimolal, at least 80 millimolal, or at least 100 millimolal, and may, for example, be present at no more than 500 millimolal, 400 millimolal, 300 millimolal, 200 millimolal, 150 millimolal, or 100 millimolal.

In some embodiments, the phytic acid is 10-100 millimolal, 20-90 millimolal, 30-80 millimolal, or 40-70 millimolal.

In some embodiments, the recovered activity of the phytase is at least 15%, at least 17%, at least 19%, at least 20%, at least 23%, at least 25%, at least 27%, or at least 30% higher, after admixing the granule with at least one food or animal feed ingredient and pelleting the resulting admixture with steam treatment, as compared to a control phytase not stabilized by phytic acid.

The phytase may be phytase BP17, e.g. phytase BP17 produced in a *Trichoderma* host cell that comprises a deletion of the endo-N-acetyl glucosaminidase gene.

The invention further provides a pellet composition comprising a granule composition as described.

The invention further provides a method for producing a heat-treated food or animal feed pellet comprising the steps of: admixing a granule composition as described with at least one food or animal feed ingredient; and pelleting the resulting admixture with steam.

## DESCRIPTION OF THE FIGURES

The present teachings will be described by reference to the following Figures:

Fig. 1 shows the recovered phytase activity obtained after pelleting at 90°C or 95°C relative to the phytase activity of the mash before pelleting.

Fig. 2 shows a Differential Scanning Calorimetry plot of DSC Tm for 0.5mg/ml phytase with and without 15mM phytic acid.

Fig. 3 shows a Differential Scanning Calorimetry plot of DSC Tm for 0.5mg/ml phytase with and without 15mM inositol.

Fig. 4 shows a plot of DSC Tm versus concentration of phytic acid. (0.5mg/ml phytase.)

Fig. 5 shows a plot of DSC Tm versus concentration of phytic acid, comparing BP17 EDT to Ronozyme P-(CT), a commercially available phytase from DSM.

## SEQUENCES

SEQ ID NO: 1= BP17, a variant phytase of *Buttiauxella sp.* comprising 12 amino acid substitutions compared to the wild type (SEQ ID NO:4), lacking the signal sequence (SEQ ID NO:12)

SEQ ID NO: 2 = BP11, a variant phytase of *Buttiauxella sp.* comprising 11 amino acid substitutions compared to the wild type (SEQ ID NO: 4), lacking the signal sequence (SEQ ID NO: 12).

SEQ ID NO: 3 = BP111, a variant phytase of *Buttiauxella sp.* comprising 21 amino acid substitutions compared to the wild type (SEQ ID NO:4), lacking the signal sequence (SEQ ID NO: 12).

SEQ ID NO: 4 = wild type phytase encoded by *Buttiauxella sp.* strain P 1-29 deposited under accession number NCIMB 41248, lacking the signal sequence (SEQ ID NO: 12).

SEQ ID NO: 5 = BP17 with an additional amino acid substitution E121T.

SEQ ID NO: 6 = BP17 with an additional amino acid substitution P394N.

SEQ ID NO: 7 = BP17 with an additional amino acid substitution D386N.

SEQ ID NO: 8 = BP17 with additional amino acid substitutions K202N and N204T.

SEQ ID NO: 9 = BP17 with additional amino acid substitutions Q151N and P153S.

SEQ ID NO: 10 = BP17 with an additional amino acid substitution P373T.

SEQ ID NO: 11 = BP17 with an additional amino acid substitution Q76N.

SEQ ID NO: 12 = signal sequence for wild type (SEQ ID NO: 4).

## DETAILED DESCRIPTION

The practice of the present teachings will employ, unless otherwise indicated, conventional techniques of molecular biology (including recombinant techniques), microbiology, cell biology, biochemistry, and animal feed pelleting, which are within the skill of the art. Such techniques are explained fully in the literature, for example, Molecular Cloning: A Laboratory Manual, second edition (Sambrook et al., 1989); Oligonucleotide Synthesis (M. J. Gait, ed., 1984; Current Protocols in Molecular Biology (F. M. Ausubel et al., eds., 1994); PCR: The Polymerase Chain Reaction (Mullis et al., eds., 1994); Gene Transfer and Expression: A Laboratory Manual (Kriegler, 1990), and Fairfield, D. 1994. Chapter 10, Pelleting Cost Center. In Feed Manufacturing Technology IV. (McEllhiney, editor), American Feed Industry Association, Arlington, Va., pp. 110-139.

Unless defined otherwise herein, all technical and scientific terms used herein have the same meaning as commonly understood by one of ordinary skill in the art to which the present teachings belong. Singleton, et al., Dictionary of Microbiology and Molecular Biology, second ed., John Wiley and Sons, New York (1994), and Hale & Markham, The Harper Collins Dictionary of Biology, Harper Perennial, NY (1991) provide one of skill with a general dictionary of many of the terms used in this invention. Any methods and materials similar or equivalent to those described herein can be used in the practice or testing of the present teachings.

Numeric ranges provided herein are inclusive of the numbers defining the range.

Unless otherwise indicated, nucleic acids are written left to right in 5' to 3' orientation; amino acid sequences are written left to right in amino to carboxy orientation, respectively.

**Definitions**

As used herein, the term "phytic acid" refers to inositol hexaphosphate (IP6), but as IP6 generally doesn't exist in pure form, the term also encompasses inositol phosphates that are catabolites of IP6, including inositol pentaphosphate (IP5), inositol tetraphosphate (IP4), inositol triphosphate (IP3), inositol diphosphate (IP2), and inositol monophosphate (IP1), salts thereof, and mixtures thereof. The term "phytic acid" does not refer to inositol, the fully hydrolyzed product of phytic acid (IP0). Thus, the term "phytic acid" encompasses catabolites, hydrolysis products, salts and analogs (e.g. esters and salts). As used herein, the terms IP6, IP5, IP4, IP3, IP2, IP1, and salts thereof, refer to any isomer of that particular inositol phosphate.

While IP6, IP5, IP4, IP3, IP2, and IP1 in salt form are typically referred to as "phytates", as used herein the term "phytic acid" also includes such phytates. The phytate counterion is typically sodium (such as sodium phytate). However the counterion may be, for example, calcium, magnesium, iron or zinc.

Hence, the term "phytic acid" as used herein includes references to the acid form and the salt form of phytic acid. In certain embodiments, the phytic acid is the acid form or combinations of acid forms. In certain embodiments the phytic acid is the salt form or combinations of salt forms. In certain embodiments the phytic acid is a combination of the acid form (or combinations of acid forms) and the salt form (or combinations of salt forms).

As used herein, the term "millimolal" is used for both liquids and solids.

The term "millimolal" for solutions (liquids) is used herein as defined in the scientific literature - i.e. the molality of a solution is defined as the amount in moles of a constituent (which here is phytic acid) divided by the mass of the solvent in kilograms (which here is everything in the solution other than phytic acid). Thus, 1,000 millimolal is equivalent to 1 molal.

The term "millimolal" for solids in the present context is defined as the amount of phytic acid (in millimoles) divided by the mass of solvent of the solid (e.g. the enzyme layer of granule), wherein the solvent is defined as being the non-phytic acid solids (such as in the enzyme layer of a granule which comprises phytase and phytic acid).

A "feed" and a "food," respectively, means any natural or artificial diet, meal or the like or components of such meals intended or suitable for being eaten, taken in, digested, by a non-human animal and a human being, respectively.

As used herein, the term "food" is used in a broad sense - and covers food and food products for humans as well as food for non-human animals (i.e. a feed).

The term "feed" is used with reference to products that are fed to animals in the rearing of livestock. The terms "feed" and "animal feed" are used interchangeably. In a preferred embodiment, the food or feed is for consumption by non-ruminants and ruminants. Examples of ruminants include cows, sheep, goats and horses. Examples of non-ruminant animals include mono-gastric animals such as pigs, poultry (such as chickens and turkeys), fish (such as salmon), dogs, cats, and humans.

The food or feed may be in the form of a solution or as a solid - depending on the use and/or the mode of application and/or the mode of administration. In some embodiments, the enzymes mentioned herein may be used as - or in the preparation or production of - a food or feed substance.

As used herein the term "food or feed ingredient" includes a formulation, which is or can be added to foods or foodstuffs and includes formulations which can be used at low levels in a wide variety of products. The food ingredient may be in the form of a solution or as a solid - depending on the use and/or the mode of application and/or the mode of administration. The enzymes described herein may be used as a food or feed ingredient or in the preparation or production. The enzymes may be - or may be added to - food supplements.

Feed compositions for monogastric animals typically include composition comprising plant products which contain phytate. Such compositions include cornmeal, soybean meal, rapeseed meal, cottonseed meal, maize, wheat, barley and sorghum-based feeds. The enzymes described herein may be - or may be added to - foods or feed substances and compositions.

As used herein, the term "amino acid sequence" is synonymous with the terms "polypeptide," "protein," and "peptide," and are used interchangeably. Where such amino acid sequences exhibit activity, they may be referred to as an "enzyme." The conventional one-letter or three-letter codes for amino acid residues are used, with amino acid sequences being presented in the standard amino-to-carboxy terminal orientation (*i.e.,* N→C).

The term "nucleic acid" encompasses DNA, RNA, heteroduplexes, and synthetic molecules capable of encoding a polypeptide. Nucleic acids may be single stranded or double stranded, and may be chemical modifications. The terms "nucleic acid" and "polynucleotide" are used interchangeably. Because the genetic code is degenerate, more than one codon may be used to encode a particular amino acid, and the present compositions and methods encompass nucleotide sequences that encode a particular amino acid sequence. Unless otherwise indicated, nucleic acid sequences are presented in 5'-to-3' orientation.

By "homologue" shall mean an entity having a specified degree of identity with the subject amino acid sequences and the subject nucleotide sequences. A homologous sequence is taken to include an amino acid sequence that is at least 75%, 80%, 85%, 86%, 87%, 88%, 89%, 90%, 91%, 92%, 93%, 94%, 95%, 96%, 97%, 98% or even 99% identical to the subject sequence, using the conventional sequence alignment tool Clustal V with default parameters. Typically, homologues will include the same active site residues as the subject amino acid sequence, though may include any number of conservative amino acid substitutions. Exemplary conservative amino acid substitutions are listed in the following Table of Conservative Amino Acid Substitutions.

Conservative amino acid substitutions

| For Amino Acid | Code | Replace with any of |
|---|---|---|
| Alanine | A | D-Ala, Gly, beta-Ala, L-Cys, D-Cys |
| Arginine | R | D-Arg, Lys, D-Lys, homo-Arg, D-homo-Arg, Met, !!e, D-Met, D-Ile, Orn, D-Orn |
| Asparagine | N | D-Asn, Asp, D-Asp, Glu, D-Glu, Gln, D-Gln |
| Aspartic Acid | D | D-Asp, D-Asn, Asn, Glu, D-Glu, Gln, D-Gln |
| Cysteine | C | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, b-Ala, Acp |
| Isoleucine | I | D-Ile, Val, D-Val, Leu, D-Leu, Met, D-Met |
| Leucine | L | D-Leu, Val, D-Val, Leu, D-Leu, Met, D-Met |
| Lysine | K | D-Lys, Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-I-thioazolidine-4- carboxylic acid, D-or L-1-oxazolidine-4-carboxylic acid |
| Serine | S | D-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Ile, D-Ile, Met, D-Met |

As used herein, the term "phytase" means a protein or polypeptide which is capable of catalysing the hydrolysis of esters of phosphoric acid, including phytate/phytic acid, and releasing inorganic phosphate. Illustrative phytases are discussed and referenced throughout the present teachings, and includes E. Coli phytase (US Patent 6,110,719), and those obtained from any of a variety of sources, including *Ascomycetes, Aspergillus awamori, Aspergillus niger, Thermomyces, Humicola, Basidiomycetes, Bacillus subtilis, and Schwannniomyces occidentalis.* Some phytases in addition to phytate are capable of hydrolysing at least some of the inositol-phosphates of intermediate degrees of phosphorylation. In one embodiment, the active enzyme produced or modified in the present teachings is a 6-phytase. The 6-phytase is also called "4-phytase" or "phytate 6-phosphatase". Further phytases include histidine acid phytases (HAP), which is a group comprising members found among prokaryotes (e.g. appA phytase from *Escherichia coli*) and eukaryotes (phyA and B from *Aspergillus* sp., HAP phytases from yeast and plants. HAP phytases share a common active site motif, RHGXRXP, at the N-terminal end and a HD motif at the C-terminal end in their DNA sequences.

The present phytases may be "precursor," "immature," or "full-length," in which case they include a signal sequence, or "mature," in which case they lack a signal sequence. Mature forms of the polypeptides are generally the most useful. Unless otherwise noted, the amino acid residue numbering used herein refers to the mature forms of the respective phytase polypeptides. The present amylase polypeptides may also be truncated to remove the N or C-termini, so long as the resulting polypeptides retain phytase activity.

The terms, "wild-type," "parental," or "reference," with respect to a polypeptide, refer to a naturally-occurring polypeptide that does not include a man-made substitution, insertion, or deletion at one or more amino acid positions. Similarly, the terms "wild-type," "parental," or "reference," with respect to a polynucleotide, refer to a naturally-occurring polynucleotide that does not include a man-made nucleoside change. However, note that a polynucleotide encoding a wild-type, parental, or reference polypeptide is not limited to a naturally-occurring polynucleotide, and encompasses any polynucleotide encoding the wild-type, parental, or reference polypeptide.

The term "variant," with respect to a polypeptide, refers to a polypeptide that differs from a specified wild-type, parental, or reference polypeptide in that it includes a man-made substitution, insertion, or deletion at one or more amino acid positions. Similarly, the term "variant," with respect to a polynucleotide, refers to a polynucleotide that differs in nucleotide sequence from a specified wild-type, parental, or reference polynucleotide. The identity of the wild-type, parental, or reference polypeptide or polynucleotide will be apparent from context.

A "vector" refers to a polynucleotide sequence designed to introduce nucleic acids into one or more cell types. Vectors include cloning vectors, expression vectors, shuttle vectors, plasmids, phage particles, cassettes and the like.

An "expression vector" refers to a DNA construct comprising a DNA sequence encoding a polypeptide of interest, which coding sequence is operably linked to a suitable control sequence capable of effecting expression of the DNA in a suitable host. Such control sequences may include a promoter to effect transcription, an optional operator sequence to control transcription, a sequence encoding suitable ribosome binding sites on the mRNA, enhancers and sequences that control termination of transcription and translation.

As used herein, the term "expression" refers to the process by which a polypeptide is produced based on a nucleic acid sequence. The process includes both transcription and translation.

A "promoter" refers to a regulatory sequence that is involved in binding RNA polymerase to initiate transcription of a gene. The promoter may be an inducible promoter or a constitutive promoter. A non-limiting example of an inducible promoter which may be used is *Trichoderma reesei* cbh1, which is an inducible promoter.

The term "operably linked" means that specified components are in a relationship (including but not limited to juxtaposition) permitting them to function in an intended manner. For example, a regulatory sequence is operably linked to a coding sequence if the expression of the coding sequence is under control of the regulatory sequences..

A "host strain" or "host cell" is an organism into which an expression vector, phage, virus, or other DNA construct, including a polynucleotide encoding a polypeptide of interest (e.g., a phytase) has been introduced. Exemplary host strains are Trichoderma sp. The term "host cell" includes protoplasts created from cells.

The term "recombinant," when used in reference to a subject cell, nucleic acid, protein or vector, indicates that the subject has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell, or express native genes at different levels or under different conditions than found in nature.

A "signal sequence" (also termed "presequence," "signal peptide," "leader sequence," or "leader peptide") refers to a sequence of amino acids bound to the N-terminal portion of a protein which facilitates the secretion of the mature form of the protein from the cell (e.g. SEQ ID NO: 12). The signal sequence targets the polypeptide to the secretory pathway and is cleaved from the nascent polypeptide once it is translocated in the endoplasmic reticulum membrane. The mature form of the extracellular protein (e.g. SEQ ID NO: 1) lacks the signal sequence which is cleaved off during the secretion process.

A "selective marker" or "selectable marker" refers to a gene capable of being expressed in a host to facilitate selection of host cells carrying the gene. Examples of selectable markers include but are not limited to antimicrobials (e.g., hygromycin, bleomycin, or chloramphenicol) and/or genes that confer a metabolic advantage, such as a nutritional advantage on the host cell. The term "derived from" encompasses the terms "originated from," "obtained from," "obtainable from," "isolated from," and "created from."

"Filamentous fungi" refers to all filamentous forms of the subdivision Eumycotina and Oomycota (See, Alexopoulos, C. J. (1962), Introductory Mycology, Wiley, New York). These fungi are characterized by a vegetative mycelium with a cell wall composed of chitin, cellulose, and other complex polysaccharides. The filamentous fungi of the present teachings are morphologically, physiologically, and genetically distinct from yeasts. Vegetative growth by filamentous fungi is by hyphal elongation and carbon catabolism is obligatorily aerobic. In the present teachings, the filamentous fungal parent cell may be a cell of a species of *Trichoderma, e.g., Trichoderma reesei* (previously classified as *T. longibrachiatum* and currently also known as *Hypocrea jecorina*), *Trichoderma viride, Trichoderma koningii,* or *Trichoderma harzianum.* Additional filamentous fungi include Aspergillus, Fusarium, Chrysosporium, Penicillium, Humicola, Neurospora, Myceliophthora, or alternative sexual forms thereof such as Emericella, Hypocrea.

The term "culturing" refers to growing a population of microbial cells under suitable conditions for growth, in a liquid or solid medium.

The term "heterologous" with reference to a polynucleotide or protein refers to a polynucleotide or protein that does not naturally occur in a host cell.

The term "endogenous" with reference to a polynucleotide or protein refers to a polynucleotide or protein that occurs naturally in the host cell.

As used herein, the terms "transformed," "stably transformed," and "transgenic," used with reference to a cell means that the cell contains a non-native (e.g., heterologous) nucleic acid sequence integrated into its genome or carried as an episome that is maintained through multiple generations.

The term "introduced" in the context of inserting a nucleic acid sequence into a cell, means "transfection", "transformation" or "transduction," as known in the art.

The terms "isolated" and "separated" refer to a compound, protein (polypeptides), cell, nucleic acid, amino acid, or other specified material or component that is removed from at least one other material or component with which it is naturally associated as found in nature.

As used herein, the term "purified" refers to material (e.g., an isolated polypeptide or polynucleotide) that is in a relatively pure state, e.g., at least about 90% pure, at least about 95% pure, at least about 98% pure, or even at least about 99% pure.

As used herein, the terms "modification" and "alteration" are used interchangeably and mean to change or vary. In the context of modifying or altering a polypeptide, these terms may mean to change the amino acid sequence, either directly or by changing the encoding nucleic acid, or to change the structure of the polypeptide such as by glycosylating the enzyme.

The term "glycosylation" as used herein refers to the attachment of glycans to molecules, for example to proteins. Glycosylation may be an enzymatic reaction. The attachment formed may be through covalent bonds. The phrase "highly glycosylated" refers to a molecule such as an enzyme which is glycosylated at all or nearly all of the available glycosylation sites, for instance N-linked glycosylation sites.

The term "glycan" as used herein refers to a polysaccharide or oligosaccharide, or the carbohydrate section of a glycoconjugate such as a glycoprotein. Glycans may be homo- or heteropolymers of monosaccharide residues. They may be linear or branched molecules.

As used herein, the term "solid support" refers to an inert solid material on to which the phytase and phytic acid is sprayed. Optionally other materials - such as enzymes, water soluble agents, dispersible agents, and other components suitable for granules - can also be sprayed onto the solid support. Examples of solid supports include, but are not limited to, sodium sulphate, magnesium sulphate, granulated sucrose, starch-sucrose non-pareils (ASNP) and maltodextrin.

As used herein, the term "granule" refers to a core which may or may not have coating layer(s).

As used herein, the term "multi-layered granule" refers to a composition comprising a core and at least one coating layer.

The term "core" as used herein is interchangeable with the term "seed".

The term "coating layer" and "layer" as used herein are interchangeable. The coating layer(s) generally encapsulates the core in order to form a substantially continuous layer so that the core surface has few or no uncoated areas. The materials (e.g. the agents, components and enzyme detailed herein) used in the granule and/or multi-layered granule are suitable for the use in foods and/or animal feeds. The materials can be food grade or feed grade.

The term "outer coating layer" as used herein refers to the coating layer of the multi-layered granule which is the furthest from the core (i.e. the last coating layer which is applied).

As used herein, the term "enzyme coating layer" refers to an enzyme layer which comprises at least one enzyme. In some embodiments the enzyme layer comprises at least two enzymes. In some embodiments, the enzyme layer comprises at least three enzymes.

"NCIMB" is the name of a depository for organisms located in Aberdeen, Scotland named The National Collection of Industrial, food, and Marine Bacteria.

As used herein, the terms "pellets" and "pelleting" refer to solid, rounded, spherical and cylindrical tablets or pellets and the processes for forming such solid shapes, particularly feed pellets and solid, extruded animal feed. Known food and animal feed pelleting manufacturing processes generally include admixing together food or feed ingredients for about 1 to about 5 minutes at room temperature, transferring the resulting admixture to a surge bin, conveying the admixture to a steam conditioner, optionally transferring the steam conditioned admixture to an expander, transferring the admixture to the pellet mill or extruder, and finally transferring the pellets into a pellet cooler. Fairfield, D. 1994. Chapter 10, Pelleting Cost Center. In Feed Manufacturing Technology IV. (McEllhiney, editor), American Feed Industry Association, Arlington, Va., pp. 110-139.

As used herein, the term "heat-treated food or animal feed pellets" refers to unpelleted admixtures which are subjected to a heat treatment (such as steam conditioning), typically at a temperature of at least 90°C for at least 30 seconds (such as for example 30 seconds at 90°C and/or 30 seconds at 95°C). The admixture can then be extruded to form the animal feed pellets.

As used herein, the term "stability" refers to any of a variety of effects in which the enzymatic activity or other functional property of a phytase enzyme is beneficially maintained or improved. A phytase can exhibit stability by showing any of improved "recovered activity," "thermostability", and/or "inactivity reversibility."

As used herein, the term "recovered activity" refers to the ratio of (i) the activity of a phytase after a treatment involving one or more of the following stressors: heating, increased pressure, increased pH, decreased pH, storage, drying, exposure to surfactant(s), exposure to solvent(s), and mechanical stress) to (ii) the activity of the phytase before the treatment. The recovered activity may be expressed as a percentage.

The percent recovered activity is calculated as follows:

$$\% \text{ recovered activity} = \left( \frac{\text{activity after treatment}}{\text{activity before treatment}} \right) \times 100 \%$$

In the context of pelleting experiments, the "activity before treatment" can be approximated by measuring the phytase activity present in the mash that does not undergo treatment in a manner that is otherwise matched to the phytase that does undergo treatment. For example, the phytase in the untreated mash is handled and stored for a similar time and under similar conditions as the phytase in the treated mash, to control for possible interactions or other effects outside of the specified treatment per se.

As used herein, the term "control phytase" in conjunction with a phytase, refers to the same kind of phytase molecule (e.g. BP17 phytase) as that which has a stabilizing agent added (e.g.-phytic acid is present), except that the stabilizing agent was lacking. For example, in one sample phytase BP17 is stabilized with phytic acid and in the comparative control phytase sample the only difference is that phytase BP17 has not been stabilized with phytic acid, such a phytase being a "control phytase".

As used herein, a "unit of phytase activity" is the amount of enzyme which is able to release 1 $\mu$mol phosphate per minute. Phytase activity is assayed according to AOAC (Association of Analytical Chemists) Official Method 2000.12, as described in "Determination of phytase activity in feed by a colorimetric enzymatic method: collaborative interlaboratory study" Engelen A J, van der Heeft F C, Randsdorp P H, Somers W A, Schaefer J, van der Vat B J. J AOAC Int. 2001 May-June; 84(3):629-33. Briefly, the ground samples are extracted in 220mM sodium acetate trihydrate, 68.4 mM calcium chloride dehydrate, 0.01% Tween 20, pH 5.5. The supernatant is then assayed. The assay measures the release of inorganic phosphate from rice phytase, at pH 5.5, for 60 min at 37C. The assay is stopped with acidic molybdate/vanadate reagent, and phosphate is quantified by intensity of yellow colored complex of the vanadomolybdophosphor.

As used herein, melting temperature (Tm) of the enzyme can be measured by techniques known in the art. One suitable technique is differential scanning calorimetry (DSC). Other suitable techniques for determining melting temperatures include light scattering, circular dichroism and enzymology experiments. DSC is a thermoanalytical technique. It is used to measure the amount of energy required to maintain the temperature of a sample at any one temperature across a range of temperature. As the tested temperature increases, a sample eventually reaches its melting temperature. The melting temperature Tm is observed as an endothermic peak in the DSC graph. As used herein, the term "DSC Tm" refers to the melting temperature of the enzyme as measured by DSC at a scan rate of 90C/min and a solution pH of 5.5.

The following hypothetical experiment is provided to further illustrate and explain the terminology of the present teachings. Here, the units in the table are for convenience depicted as commencing with 100 arbitrary units of phytase activity pre-pelleting. First, looking at the top row of data, for a given phytase composition such as BP17 lacking phytic acid, the "percent recovered activity" is 50% after the pelleting treatment. Second, looking at the bottom row of data, for a given phytase composition such as BP17 with phytic acid, the "percent recovered activity" of the phytase after the pelleting treatment is 80%. Third, looking at the far right column, BP phytase with phytic acid has a phytase activity that is 60% higher (80-50=30; 30/50=.6=60%) as compared to the "control phytase" i.e. phytase that was not stabilized by phytic acid. In some embodiments, these improvements can be accompanied by improvements in thermostability, for example Tm as measured by DSC Tm. In some embodiments, these improvements can be accompanied by improvements in inactivity reversibility (that is, the ability of lost activity to be recovered after a period of time).

| | Pre-Pelleting | Post-Pelleting |
|---|---|---|
| Control phytase (No Phytic Acid) | 100 units phytase activity | 50 units phytase activity |
| Stabilized phytase (With Phytic Acid) | 100 units phytase activity | 80 units phytase activity |

**EXEMPLARY EMBODIMENTS**

The present teachings relate to the surprising observation that the thermal stabilization of a phytase enzyme after pre-incubation with molar excess of its substrate or its potential substrate analog results in stability of the enzyme. More specifically, the present teachings provide that the stability of phytase can be improved including phytic acid in functional proximity to the phytase. Further, the present teachings provide an excess of phytic acid can improve the stability of phytase during food and animal feed processing and can result in heat-treated food and animal feed pellets which have improved phytase activity.

**Phytic acid**

In some embodiments, the phytic acid comprises the unhydrolyzed inositol phosphate, IP6. Typically, commercial sources of phytic acid are not pure IP6. In some embodiments, the phytic acid is a mixture of partially hydrolyzed inositol phosphates (e.g. IP6, IP5, IP4, IP3, IP2 and IP1). Thus, useful types of phytic acid are phytic acid catabolites, hydrolysis products, salts and analogs (e.g. esters and salts). Inositol (the fully hydrolyzed product of phytic acid - IP0) may be present in the source of phytic acid for use in the present teachings but we have found that under some conditions inositol on its own does not stabilize phytase. Thus, in accordance with the present teachings inositol phosphates are useful for the stabilization of phytase in feed or food composition.

The phytic acid for use in the present teachings may be chemically synthesized. In addition or alternatively, the phytic acid for use in the present teachings may be present in or derived from a food, animal feed or ingredients thereof. Phytic acid is found in the hulls of nuts, seeds and grains. Sources of phytic acid include, but are not limited to, linseeds, sesame seeds, almonds, brazil nuts, coconuts, peanuts, walnuts, maize (corn), oats, oat meal, rice, wheat, beans, chickpeas, soybeans, tofu and potatoes. Typically, phytic acid is obtained from grains (such as corn, wheat or rice) though extraction into water.

The phytic acid which is used for stabilizing phytase is in addition to the amount of phytic acid which may inherently be present in the ingredients of a food or animal feed which the phytase (such as in the form of a multi-layered granule) is admixed with before pelleting. In other words, the phytic acid which is used for stabilizing phytase is an exogenous phytase; the phytic acid present in the ingredients of a food or animal feed is an endogenous phytic acid. Accordingly, the calculated greater than 10 millimolal phytic acid provided by the present teachings, and other stated millimolal levels for stabilizing phytase provided by the present teachings, does not include trace amounts of endogenous phytic acid.

**Phytase**

As used herein, the term "phytase" means a protein or polypeptide which is capable of catalyzing the hydrolysis of esters of phosphoric acid, including phytate and phytic acid, and releasing inorganic phosphate. Some phytases in addition to phytate are capable of hydrolyzing at least some of the inositol-phosphates of intermediate degrees of phosphorylation.

The term "phytase" may be one phytase or a combination of phytases unless the context clearly dictates otherwise.

Phytase enzymes, such as e.g. the 6-phytase BP17 derived from *Buttiauxella sp.,* are added to foods and animal feeds to increase phosphate availability thus increasing the nutritional value of the product. The processing of the food or animal feed, for example under heat and high pressure, can denature the phytase and reduce its activity.

The phytase used in the present teachings may be any phytase which is suitable for use in foods or animal feeds.

In one embodiment, the active enzyme produced or modified in the current description is a phytase, more in particular a 6-phytase.

The 6-phytase is also called "4-phytase" or "phytate 6-phosphatase". Further phytases include histidine acid phytases (HAP), which is a group comprising members found among prokaryotes (e.g. appA phytase from *Escherichia coli*) and eukaryotes (phyA and B from *Aspergillus* sp., HAP phytases from yeast and plants. HAP phytases share a common active site motif, RHGXRXP, at the N-terminal end and a HD motif at the C-terminal end in their DNA sequences. This allows a two-step mechanism in the hydrolysis of phosphomonoesters. The phyA and phyB from *A. niger* and appA from *E. coli* are the representatives which have been the most characterized.

In one embodiment, the phytase is 6-phytase, an *E. coli* phytase, an *Aspergillus* phytase, or Ronozyme phytase (DSM Corporation).

In a highly preferred embodiment, the active enzyme to be produced or modified is BP17. BP17 is an enzyme variant of a *Buttiauxella sp.* phytase. The sequence for BP17 (excluding signal peptide), which is used as a reference for position numbering of amino acids throughout, is shown as SEQ ID No. 1.

In another embodiment, the active enzyme to be is produced or modified BP11. BP11 is an enzyme variant of a *Buttiauxella sp.* phytase. The sequence for BP11 (excluding signal peptide) is shown as SEQ ID No. 2.

In another embodiment, the active enzyme to be produced or modified is BP111. BP111 is an enzyme variant of a *Buttiauxella sp.* phytase. The sequence for BP111 (excluding signal peptide) is shown as SEQ ID No. 3.

All of these phytases are variants of the wild-type sequence such as that derived from *Buttiauxella sp.* strain P 1-29 deposited under accession number NCIMB 41248, having the sequence is shown as SEQ ID No. 4.

The above detailed phytase enzymes are mature proteins lacking a signal sequence. The appropriate signal sequence derived from *Buttiauxella sp.* strain P 1-29 deposited under accession number NCIMB 41248, is shown as SEQ ID No. 12.

In one embodiment, the phytase is produced in a *Trichoderma* host cell. In some embodiments, the phytase is produced in a *Trichoderma* host cell that comprises a deletion of the endo-N-acetyl glucosaminidase gene. The endo-N-acetyl glucosaminidase gene encodes the enzyme endo-N-acetyl glucosaminidase which removes N-linked glycosylation from other proteins such as phytases. In some embodiments, the phytase is phytase BP17 produced in a *Trichoderma* host cell that comprises a deletion of the endo-N-acetyl glucosaminidase gene. Exemplary strains are taught for example in WO 09/114380, see for instance Example 5.

The amount of Phytase Units added to the food or animal feed will depend on the composition of the food or feed itself. Foods and feeds containing lower amounts of available phosphorous will generally require higher amounts of phytase activity. The amount of phytase required may be determined by the skilled person.

In one embodiment, the amount of phytase incorporated into in a food or animal feed may be between 0.1 to 20 Units Phytase activity per gram of the food or animal feed. Typically, the amount of phytase is about 50 grams (12,000 U/g phytase granule) per ton (1,000kg) of food or animal feed; this is equivalent to 0.6 units phytase per gram of food or animal feed.

**Phytase and Phytic acid**

The phytase and/or phytic acid are in a granule composition, such as a multi-layered granule. The phytase and the phytic acid are in adjacent coating layers of a multi-layered granule, or phytase is in the core of the granule and phytic acid is in the adjacent coating layer.

In one embodiment of the present teachings, there are at least 3 moles of phytic acid for every mole of phytase.

**Stabilized phytase**

The stability of a phytase may be improved in the liquid state and/or in the solid state compared to that of the control phytase which has not been stabilized by the use of phytic acid.

The phytase is stabilized by at least 10 millimolal of phytic acid. In some embodiments, phytase is stabilized by at least 20, at least 50, at least 100 millimolal, or at least 150 millimolal of phytic acid.

In another embodiment, phytase is stabilized by phytic acid in the range of 10 millimolal to 150 millimolal. In other embodiments, phytase is stabilized by phytic acid in the range of 10 millimolal to 100 millimolal. In some embodiments, phytase is stabilized by phytic acid in the range of 20 millimolal to 150 millimolal. In other embodiments, phytase is stabilized by phytic acid in the range of 20 millimolal to 100 millimolal. In some embodiments, phytase is stabilized by phytic acid in the range of 50 millimolal to 150 millimolal. In other embodiments, phytase is stabilized by phytic acid in the range of 50 millimolal to 100 millimolal. In some embodiments, phytase is stabilized by phytic acid in the range of 100 millimolal to 150 millimolal.

In some embodiments, the phytic acid is present at less than 5%, 2%, 1%, .5%, .1%, .05%, .01%, .005%, .001% weight/weight, expressed as weight of phytic acid to weight on an entire granule in which the phytic acid resides, including layers, if present, that lack phytic acid.

In further embodiments, the molar ratio of phytic acid and phytase is 1:1, 2:1, 3:1, 5:1, 10:1, 20:1, 25:1 or 50:1.

In one embodiment, after treatment (e.g. heating, increased pressure, increased pH, decreased pH, storage, drying, exposure to surfactant(s), exposure to solvent(s), and/or mechanical stress) the stabilized phytase has a phytase activity which is at least 10%, at least 15%, at least 20%, at least 25%, at least 30%, at least 40%, at least 50%, or at least 60% higher than that of the control phytase which has not been stabilized by the use of phytic acid. In one embodiment, after treatment (e.g. heating, increased pressure, increased pH, decreased pH, storage, drying, exposure to surfactant(s), exposure to solvent(s), and/or mechanical stress) the stabilized phytase has a phytase activity which is 10%-60% higher than that of the control phytase which has not been stabilized by phytic acid.

In one embodiment, the enzymatic activity of a stabilized phytase after treatment (e.g. heating, increased pressure, increased pH, decreased pH, storage, exposure to surfactant(s), exposure to solvent(s), and/or mechanical stress) is at least 65%, at least 70%, at least 75%, at least 80%, at least 85%, at least 90%, or at least 95% that of the stabilized phytase before the treatment.

In one embodiment, at least 65%, at least 70%, at least 75%, at least 80%, at least 85%, at least 90%, or at least 95% phytase activity is recovered.

In one embodiment, at least 65%, at least 70%, at least 75%, at least 80%, at least 85%, at least 90%, or at least 95% phytase activity is recovered in the solid state during food or animal feed pelleting.

In one embodiment, at least 65%, at least 70%, at least 75%, at least 80%, at least 85%, at least 90%, or at least 95% phytase activity is recovered after treatment (e.g. heating, increased pressure, increased pH, decreased pH, storage, exposure to surfactant(s), exposure to solvent(s), and/or mechanical stress). For example, at least 80% phytase activity is recovered after treatment.

In one embodiment, at least 65%, at least 70%, at least 75%, at least 80%, at least 85%, at least 90%, or at least 95% phytase activity is recovered after food or animal feed pelleting. For example, at least 80% phytase activity is recovered after food or animal feed pelleting.

In some embodiments, at least 65%, at least 70%, at least 75%, at least 80%, at least 85%, at least 90%, or at least 95% phytase activity is recovered in the solid state after food or animal feed pelleting. For example, at least 80% phytase activity is recovered in the solid state after food or animal feed pelleting.

In one embodiment, the recovered activity of a phytase stabilized by phytic acid is improved by at least 15%, at least 17%, at least 19%, at least 20%, at least 23%, at least 25%, at least 27%, at least 30%, at least 32%, at least 35%, at least 40%, at least 42%, or at least 45% compared to the control phytase not stabilized by phytic acid. For example, the recovered activity of a phytase stabilized by phytic acid is improved by at least 23% compared to the control phytase not stabilized by phytic acid.

In one embodiment, the recovered activity of a phytase stabilized by phytic acid in the solid state (such as a multi-layered granule) is improved by at least 15%, at least 17%, at least 19%, at least 20%, at least 23%, at least 25%, at least 27%, at least 30%, at least 32%, at least 35%, at least 40%, at least 42%, or at least 45% compared to the control phytase not stabilized by at least 10 millimolal phytic acid at the same conditions. For example, the recovered activity of a phytase stabilized by phytic acid in the solid state (such as a multi-layered granule) is improved by at least 32% compared to the control phytase not stabilized by at least 10 millimolal phytic acid at the same conditions.

In one embodiment, the recovered activity of a phytase stabilized by phytic acid in the solid state (such as a multi-layered granule) is improved by at least 15%, at least 17%, at least 19%, at least 20%, at least 23%, at least 25%, at least 27%, at least 30%, at least 32%, at least 35%, at least 40%, at least 42%, or at least 45% compared to the control phytase not stabilized by phytic acid in the range between 10 millimolal and 150 millimolal.

In one embodiment, the recovered activity of the stabilized phytase is improved by at least 15%, at least 17%, at least 19%, at least 20%, at least 23%, at least 25%, at least 27%, at least 30%, at least 32%, at least 35%, at least 40%, at least 42%, or at least 45% after admixing said stabilized phytase with at least one food or animal feed ingredient and treating the resulting admixture with, for example, heat (such as steam), increased pressure, increased pH, decreased pH, storage, exposure to surfactant(s), exposure to solvent(s), and/or mechanical stress, compared to the recovered activity of the control phytase not stabilized by phytic acid at a concentration of least 10 millimolal at the same conditions. For example, the recovered activity of the stabilized phytase is improved by at least 32% after admixing said stabilized phytase with at least one food or animal feed ingredient and treating the resulting admixture with, for example, heat (such as steam), increased pressure, increased pH, decreased pH, storage, exposure to surfactant(s), exposure to solvent(s), and/or mechanical stress, compared to the recovered activity of a control phytase not stabilized by phytic acid at a concentration of least 10 millimolal at the same conditions.

In another embodiment the recovered activity of the stabilized phytase is improved by at least 15%, at least 17%, at least 19%, at least 20%, at least 23%, at least 25%, at least 27%, at least 30%, at least 32%, at least 35%, at least 40%, or at least 42%, at least 45% after admixing said stabilized phytase with at least one food or animal feed ingredient and treating the resulting admixture with, for example, heat (such as steam), increased pressure, increased pH, decreased pH, storage, exposure to surfactant(s), exposure to solvent(s), and/or mechanical stress, compared to the recovered activity of a control phytase not stabilized by phytic acid at a concentration in the range between 10 millimolal and 150 millimolal at the same conditions.

In one embodiment, at least 10 millimolal phytic acid is used for stabilizing phytase in a food or feed pellet which is subjected to or has been subjected to one or more of the following treatments: heating, increased pressure, increased pH, decreased pH, storage, exposure to surfactant(s), exposure to solvent(s), and mechanical stress.

**Melting temperature (Tm)**

One way to determine the thermal stability of an enzyme is to determine the melting temperature (Tm) of the enzyme.

The melting temperature (Tm) of the enzyme can be measured by techniques known in the art. One suitable technique is differential scanning calorimetry. Other suitable techniques for determining melting temperatures include light scattering, circular dichroism and enzymology experiments.

In one embodiment, the Tm (such as the DSC Tm) of a phytase (stabilized by at least 10 millimolal phytic acid)

is increased by at least 1C, at least 1.3C, at least 1.4C, at least 1.5C, at least 2C, at least 4C, at least 6C, at least 7C, at least 7.5C, at least 7.6C, or at least 8C, compared to a control phytase.

In one embodiment, the Tm (such as the DSC Tm) of a phytase stabilized by at least 15 millimolal phytic acid is increased by at least 1C, at least 1.3C, or at least 1.4C, compared to a control phytase which is not stabilized by at least 10 millimolal phytic acid. For example, the Tm (such as the DSC Tm) of a phytase stabilized by at least 15 millimolal phytic acid is increased by at least 1.4C, compared to a control phytase which is not stabilized by at least 10 millimolal phytic acid.

In one embodiment, the Tm (such as the DSC Tm) of a phytase stabilized by at least 10 millimolal phytic acid is increased by at least 4C, at least 6C, at least 7C, at least 7.5C, at least 7.6C, or at least 8C, compared to a control phytase which is not stabilized by at least 10 millimolal phytic acid. For example, the Tm (such as the DSC Tm) of a phytase stabilized by at least 10 millimolal phytic acid is increased by at least 7.6C, compared to a control phytase which is not stabilized by at least 10 millimolal phytic acid.

In one embodiment, the $T_m$ (such as the DSC Tm) of a phytase (stabilized by phytic acid in the range between 10 millimolal and 150 millimolal) is increased by at least 1C, at least 1.3C, at least 1.4C, at least 1.5C, at least 2C, at least 4C, at least 6C, at least 7C, at least 7.5C, at least 7.6C, or at least 8C, compared to a control phytase which is not stabilized by phytic acid in the range between 10 millimolal and 150 millimolal.

**Granule and multi-layered granule**

Cores, granules and multi-layered granules may be produced by a variety of fabrication techniques including: rotary atomization, wet granulation, dry granulation, spray drying, disc granulation, extrusion, pan coating, spheronization, drum granulation, fluid-bed agglomeration, high-shear granulation, fluid-bed spray coating, crystallization, precipitation, emulsion gelation, spinning disc atomization and other casting approaches, and prill processes. Such processes are known in the art and are described in US Pat. No. 4689297 and US Pat. No. 5324649 (fluid bed processing); EP656058B1 and US Pat. No. 454332 (extrusion process); US Pat. No. 6248706 (granulation, high-shear); and EP804532B1 and US Pat. No. 6534466 (combination processes utilizing a fluid bed core and mixer coating).

The core is the inner nucleus of the multi-layered granule or is the granule. The materials used in the core can be suitable for the use in foods and/or animal feeds. US20100124586, WO9932595, and US5324649 detail suitable materials for the core.

In one embodiment, the core comprises one or more water soluble or dispersible agent(s). Suitable water soluble agents include, but are not limited to, inorganic salts (e.g. sodium sulphate, sodium chloride, magnesium sulphate, zinc sulphate, and ammonium sulphate), citric acid, sugars (e.g. sucrose, lactose, glucose, granulated sucrose, maltodextrin and fructose), plasticizers (e.g. polyols, urea, dibutyl phthalate, and dimethyl phthalate), fibrous material (e.g. cellulose and cellulose derivatives such as hydroxyl-propyl-methyl cellulose, carboxy-methyl cellulose, and hydroxyl-ethyl cellulose), phytic acid, and combinations thereof. Suitable dispersible agents include, but are not limited to, clays, nonpareils (combinations of sugar and starch; e.g. starch-sucrose non-pareils - ASNP), talc, silicates, carboxymethyl cellulose, starch, and combinations thereof.

In one embodiment, the core comprises sodium sulphate. In another embodiment, the core consists of sodium sulphate. In some embodiments, the core may comprise a phytase and/or phytic acid. In some embodiments, the core does not comprise phytase.

In some embodiments the core is coated with at least one coating layer. In one embodiment the core is coated with at least two coating layers. In another embodiment the core is coated with at least three coating layers. In a further embodiment the core is coated with at least four coating layers.

The materials used in the coating layer(s) can be suitable for use in foods and/or animal feeds. US20100124586, WO9932595, and US5324649 detail suitable materials for the coating layer.

In some embodiments, the fluid bed granulation process is employed traditionally by running and by continuously spraying one layer on top of the next layer with only a brief flushing of the lines and spray nozzles with water for cleaning purposes, without cessation in spray. In some embodiments, the granules can be made by allowing them to dry (fluidize without spray) for an additional time period (e.g. 5 minutes) after the end of each intermediate spray (for example at 70C), and an optional additional drying after the final spray can be performed (for example 20 minutes at 70C).

In some embodiments, the additional time period after the end of the intermediate spray is at least 1, 2, 3, 4, 5, 6, 7, 8, 9, 10, 11, 12, 15, 20, or 25 minutes. In some embodiments the additional time period after the end of the intermediate spray is 2-8, 3-7, or 4-6 minutes. In some embodiments, the temperature of the additional time period after the end of the intermediate spray is at least 50C, 55C, 60C, 65C, 70C, 75C, or 80C. In some embodiments, the temperature of the additional time period after the end of the intermediate step is 60C-80C or 65C-75C. In some embodiments, after the end of the intermediate spray a drying step is performed for 4-6 minutes at 65-75C.

In some embodiments, the optional drying after the final spray can be at least 5, 10, 15, 20, 25, or 30 minutes. In some embodiments, the optional drying after the final spray can be 5-30 or 10-25 minutes. In some embodiments, the temperature of the optional drying after the final spray is at least 50C, 55C, 60C, 65C, 70C, 75C, or 80C. In some embodiments, the temperature of the optional drying after the final spray is 60C-80C or 65C-75C. In some embodiments, the optional drying after the final spray is 15-25 minutes at 65C-75C.

In one embodiment, a coating layer comprises one of more of the following materials: an inorganic salt (e.g. sodium sulphate, sodium chloride, magnesium sulphate, zinc sulphate, and ammonium sulphate), citric acid, a sugar (e.g. sucrose, lactose, glucose, and fructose), a plasticizer (e.g. polyols, urea, dibutyl phthalate, and dimethyl phthalate), fibrous material (e.g. cellulose and cellulose derivatives such as hydroxyl-propyl-methyl cellulose, carboxy-methyl cellulose, and hydroxyl-ethyl cellulose), clay, nonpareil (a combination of sugar and starch), silicate, carboxymethyl cellulose, phytic acid, starch (e.g. corn starch), fats, oils (e.g. rapeseed oil, and paraffin oil), lipids, vinyl polymers, vinyl copolymers, polyvinyl alcohol (PVA), plasticizers (e.g. polyols, urea, dibutyl phthalate, dimethyl phthalate, and water), anti-agglomeration agents (e.g. talc, clays, amorphous silica, and titanium dioxide), anti-foam agents (such as Foamblast 882® and Erol 6000K®), and talc. US20100124586, WO9932595, and US5324649 detail suitable components for the coating layers.

In one embodiment, the coating layer comprises sugars, such as sucrose.

In one embodiment, the coating layer comprises a polymer such as polyvinyl alcohol (PVA).

Suitable PVA for incorporation in the coating layer(s) of the multi-layered granule include partially hydrolyzed, fully hydrolyzed and intermediately hydrolyzed having low to high degrees of viscosity.

In another embodiment, the coating layer comprises an inorganic salt, such as sodium sulphate.

In one embodiment, at least one coating layer is an enzyme coating layer. In some embodiments the core is coated with at least two enzyme layers. In another embodiment the core is coated with at least three enzyme layers.

In some embodiments, the granules of the present teachings comprise an enzyme coating layer. In some embodiments, the enzyme layer comprises at least one enzyme. In some embodiments the enzyme layer comprises at least two enzymes. In some embodiments, the enzyme layer comprises at least three enzymes. In some embodiments, the enzyme is selected from the group consisting of phytases, xylanases, phosphatases, amylases, esterases, redox enzymes, lipases, transferases, cellulases, hemi-cellulases, beta-glucanases, oxidases (e.g. hexose oxidases and maltose oxidoreductases), proteases and mixtures thereof. Generally, at least one enzyme coating layer comprises at least one phytase, and phytic acid.

In one embodiment, the enzyme coating layer comprises at least one phytase and at least one further enzyme selected from the group consisting of phytases, xylanases, phosphatases, amylases, esterases, redox enzymes, lipases, transferases, cellulases, hemi-cellulases, beta-glucanases, oxidases (e.g. hexose oxidases and maltose oxidoreductases), and proteases.

The above enzyme lists are examples only and are not meant to be exclusive. Any enzyme can be used in the granules described herein, including wild type, recombinant and variant enzymes of bacterial, fungal, yeast, plant, insect and animal sources, and acid, neutral or alkaline enzymes.

In some embodiments, the enzyme coating layer may further comprise one or more additional materials selected from the group consisting of: phytic acid, sugars (e.g. sucrose), starch (e.g. corn starch), fats, oils (e.g. rapeseed oil, and paraffin oil), lipids, vinyl polymers, vinyl copolymers, polyvinyl alcohol (PVA), plasticizers (e.g. polyols, urea, dibutyl phthalate, dimethyl phthalate, and water), anti-agglomeration agents (e.g. talc, clays, amorphous silica, and titanium dioxide), anti-foam agents (such as Foamblast 882® and Erol 6000K® available from Ouvrie PMC, Lesquin, France), and talc. US20100124586, WO9932595, and US5324649 detail suitable components for granules. Foamblast 882® is available from Emerald Foam Control, LLC. Foamblast 882® is a defoamer which is made with food grade ingredients.

In one embodiment, the multi-layered granule comprises an enzyme coating layer comprising a phytase and a coating layer comprising phytic acid that is functionally adjacent to the enzyme layer.

In one embodiment, the coating layer comprises phytic acid wherein said coating layer is functionally adjacent to a core comprising phytase and/or an enzyme coating layer comprising phytase.

In one embodiment, the outer coating layer of a multi-layered granule comprises one or more of the following coating materials: polymers (e.g. vinyl polymers, polyvinyl alcohol, and vinyl copolymers), gums, waxes, fats, oils, lipids, lecithin, pigments, lubricants, nonpareils, inorganic salts (e.g. sodium sulphate, sodium chloride, magnesium sulphate, zinc sulphate, and ammonium sulphate), talc, and plasticizers (e.g. sugars, sugar alcohols, and polyethylene glycol).

In one embodiment, the outer coating layer of a multi-layered granule comprises an inorganic salt (e.g. sodium sulphate), polyvinyl alcohol (PVA), talc or combinations thereof. In one embodiment, the outer coating layer comprises polyvinyl alcohol (PVA) and/or talc.

In one embodiment, the outer coating layer prevents or reduces the rate or extent of water, moisture or steam migration into the enzyme layer.

The multi-layered granules described herein can be produced by a variety of techniques including: fluid-bed spray-coating, pan-coating, and other techniques for building up a multi-layered granule by adding consecutive layers on top of the starting core material (the seed). See, for example, US 5324649 and US20100124586. In one embodiment, the multi-layered granules are produced using a fluid-bed spray coating process.

In one embodiment, the multi-layered granules comprise or consist of a core comprising sodium sulphate; a first coating layer comprising or consisting of phytase, sucrose, starch, phytic acid and rapeseed oil; a second coating layer comprising or consisting of sodium sulphate; and a third coating layer comprising or consisting of talc and PVA. The first coating layer is applied to the core then the second coating layer is applied to the first coating layer and then the third coating layer is applied to second coating layer.

In another embodiment, the multi-layered granules comprise or consist of a core comprising sodium sulphate; a first coating layer comprising or consisting of phytase, sucrose, starch, phytic acid and an antifoam agent (such as Foamblast 882®); a second coating layer comprising or consisting of sodium sulphate; and a third coating layer comprising or consisting of talc and PVA. The first coating layer is applied to the core then the second coating layer is applied to the first coating layer and then the third coating layer is applied to second coating layer.

**Pellets and Pelleting**

Pellets may comprise granules and/or multi-layered granules as described herein according to any of a variety of known pelleting methods, examples of which are described further below.

In one embodiment, the pellet comprises phytic acid, phytase and at least one food or feed ingredient, wherein the concentration of the phytic acid is at least 10 millimolal. In one embodiment, the pellet comprises at least one food or feed ingredient and a granule comprising phytic acid and phytase, wherein the concentration of the phytic acid in the granule is at least 10 millimolal.

Pellets of the present teachings are produced by a method in which the temperature of a feed mixture is raised to a high level by steam treatment prior to pelleting, a process known as conditioning. Subsequently, the conditioned feed mixture can be passed through a die to produce pellets of a particular size. The feed mixture can be prepared by mixing granules and/or multi-layered granules described herein with food or animal feed as described herein.

Generally, the steam conditioner treats the admixture for about 20 to about 90 seconds, and up to several minutes, at about 85C to about 95C. The amount of steam may vary in accordance with the amount of moisture and the initial temperature of the food or animal feed mix. About 4% to about 6% added steam has been reported in pelleting processes, and the amount is selected to produce less than about 18% moisture in the mash prior to pelleting, or up to about 28% moisture in mash intended for extrusion.

An optional expander process can occur for about 4 to about 10 seconds at a temperature range of about 100C to about 140C. The pellet mill portion of the manufacturing process typically operates for about 3 to about 5 seconds at a temperature of about 85C to about 95C.

Prior to pelleting, unpelleted mixtures (so-called premixes or precursors, base mixes, mash, and diluents for pellets) typically contain vitamins and trace minerals. Base mixes typically contain food and animal feed ingredients such as dicalcium phosphate, limestone, salt and a vitamin and mineral premix, but not grains and protein ingredients. Diluents include, but are not limited to, grains (for example wheat middlings and rice bran) and clays, such as phyllosilicates (the magnesium silicate sepiolite, bentonite, kaolin, montmorillonite, hectorite, saponite, beidellite, attapulgite, and stevensite). Clays also function as carriers and fluidizing agent, or diluents, for food and animal feed premixes. Mash typically comprises a complete animal diet. For example, the mash comprises or consists of corn, soybean meal, soy oil, salt, DL Methionine, limestone, dicalcium phosphate and vitamins and minerals. In one example, the mash consists of 61.10% corn, 31.43% soybean meal 48, 4% soy oil, 0.40% salt, 0.20% DL Methionine, 1.16% limestone, 1.46% dicalcium phosphate and 0.25% vitamins and minerals.

In one embodiment, a food or an animal feed is produced by admixing at least one food or animal feed ingredient (such as a mash) with a phytase and phytic acid in a granule form as described, steam conditioning the resulting admixture, followed by pelleting the admixture.

For example, an unpelleted mixture of corn meal and soy meal (such as 60% corn meal and 40% soy meal) can be admixed with multi-layered granules comprising phytase and phytic acid and then subjected to steam conditioning at 90C for 30 seconds. The admixture is then extruded to form the animal feed pellets.

In some embodiments, the compositions of the present teachings can reside in heat-treated food or animal feed pellets. Such heat-treated food or animal feed pellets can be subjected to a heat treatment (such as steam conditioning) at a temperature of at least 90C for at least 30 seconds (such as 30 seconds at 90C and/or 30 seconds at 95C). The admixture can then be extruded to form the animal feed pellets.

**Food and animal feed**

In one embodiment, the food or animal feed is a liquid such as a liquid feed. In another embodiment, the food or animal feed is a solid. As used herein, the term animal includes all animals, examples of which include of non-ruminants and ruminants (such as cows, sheep, goats and horses). Examples of non-ruminant animals include mono-gastric animals such as pigs, poultry (such as chickens and turkeys), fish (such as salmon), dogs, cats, and humans. In one embodiment, the animal feed is feed for chickens (chicken feed). In one embodiment, the animal feed is feed for pigs (pig).

The food or animal feed may comprise vegetable proteins. Vegetable proteins may be derived from legumes, oil seeds, nuts and cereals. Examples of sources of vegetable proteins include plants from the families *Fabaceae (Leguminosae), Poaceae, Cruciferaceae,* and *Chenopodiaceae.*

Suitable sources of vegetable proteins are soy beans, soy bean meal, cereals (such as maize (corn), wheat, oats, barley, rye, and sorghum), cereal meals (such as corn meal, wheat meal, oat meal, barley meal, rye meal, sorghum meal, and canola meal), brans (such as what bran, and oat bran), oil seeds (such as rapeseed and sunflower seeds), oil seed meals (such as rapeseed meal), cottonseed meal, cabbage, beet and sugar beet. These vegetable proteins are examples of food ingredients and animal feed ingredients.

The food or animal feed may comprise animal proteins. Suitable animal proteins include fish-meal and whey. The food or animal feed may comprise additives. Suitable additives include enzyme inhibitors, vitamins, trace minerals, macro minerals, coloring agents, aroma compounds, antimicrobial peptides (such as Leucocin A, Thanatin, and Tritrpticin), and enzymes.

The food or animal feed or ingredients thereof may be a liquid. The food or animal feed or ingredients thereof may be a solid. Examples include corn, wheat or soy meal.

**Enzyme Modification Embodiments**

The present teachings provide for phytases with various modifications, for example by glycosylation. In particular, the present description relates to the glycosylation of phytase. In some embodiments, the present description relates to the modification or alteration of enzymes to introduce or increase the number of glycosylation sites. This modification can be made to the polypeptide or to the encoding nucleic acid. In some embodiments the modified enzyme is a phytase and in some embodiments the enzyme is phytase BP17. The phytase BP17 amino acid sequence (SEQ ID NO: 1) contains three potential N-linked glycosylation sites according to analysis by the NetNGlyc 1.0 prediction algorithm (http://www.cbs.dtu.dk/services/NetNGlyc/). The asparagine residues that are predicted to be glycosylated are residues N169, N173 and N285 of the mature phytase BP17 sequence (lacking a signal sequence), as indicated in the SEQ ID NO: 1 below bold and underlined.

SEQ ID NO: 1

NDTPASGYQVEKVVILSRHGVRAPTKMTQTMRDVTPNTWPEWPVKLGYITPRGEHLISLMGG

FYRQKFQQQGILSQGSCPTPNSIYVWTDVAQRTLKTGEAFLAGLAPQCGLTIHHQQNLEKADP

LFHPVKAGICSMDKTQVQQAVEKEAQTPIDNLNQHYIPSLALM**N**TTL**N**FSKSPWCQKHSADKS

CDLGLSMPSKLSIKDNGNEVSLDGAIGLSSTLAEIFLLEYAQGMPQAAWGNIHSEQEWALLLKL

HNVYFDLMERTPYIARHKGTPLLQAISNALNP**N**ATESKLPDISPDNKILFIAGHDTNIANIAGMLN

MRWTLPGQPDNTPPGGALVFERLADKSGKQYVSVSMVYQTLEQLRSQTPLSLNQPAGSVQL

KIPGCNDQTAEGYCPLSTFTRVVSQSVEPGCQLQ

One embodiment of the description involves the production and use of a phytase which is highly glycosylated, for example highly glycosylated BP17, or a homologue, variant or derivative thereof. In some embodiments, the enzyme produced and/or used has more than 75%, for example more than 80%, for example more than 90% and for further example more than 95%, 96%, 97%, 98%, or 99% identity to BP17.

Some embodiments of the description involve introducing further glycosylation sites to BP17. Specifically one or more or all of the following substitutions can be introduced to BP17 (with reference to the position numbering of SEQ ID NO: 1, lacking a signal sequence) in order to introduce glycosylation sites:-E121T, P394N, D386N, K202N, N204T, Q151N and P153S, P373T, and Q76N.

In particular, a sequence of the description may comprise K202N and N204T in the same sequence. In a further embodiment of the description, a sequence of the description may comprise Q151N and P153S in the same sequence.

SEQ IN Nos: 5 to 11 present variant sequences that may be used in the present teachings (in each case the amino acid changes relative to BP17 are shown in bold and underlined) wherein amino acid substitutions have been introduced to BP17 (SEQ ID NO: 1) to increase the number of glycosylation sites.

The extent of glycosylation of an enzyme may increase the stability of the modified enzyme. Therefore some embodiments of the description provide more thermostable enzymes, for example enzymes which are more thermostable than wild-type enzymes or enzymes produced using different methods.

The present description also provides for nucleic acids encoding and capable of encoding the polypeptides of SEQ ID NO 1 and homologues, variants or derivative thereof.

The present description may also relate to the enzyme of SEQ ID NO 1 or a polypeptide derived from the parent enzyme by substitution, deletion or addition of one or several amino acids, such as 2, 3, 4, 5, 6, 7, 8, 9 amino acids, or more amino acids, such as 10 or more than 10 amino acids in the amino acid sequence of the parent protein and having the activity of the parent protein. In some embodiments this enzyme is glycosylated and in some embodiments such an enzyme has increased stability.

A further embodiment of the present description provides a method of producing an enzyme of the description, for example an enzyme wherein glycosylation is increased or the enzyme is highly glycosylated. This method involves altering the amino acid sequence of the enzyme, or altering the nucleic acid which encodes the enzyme, to increase the number of glycosylation sites. In some embodiments this alteration involves increasing the number of an N-linked glycosylation sites. In some embodiments this involves introducing the motif is Asn-Xaa-Ser/Thr to the enzyme or introducing sequence which encodes this site to the nucleic acid. In some embodiments one, or two or three or more glycosylation sites are introduced to an enzyme.

In some embodiments the method of the description is carried out on a phytase, for example BP17 (SEQ ID NO: 1). In some embodiments the method of the description introduces one or more of the following amino acid mutations in order to increase the number of glycosylation sites in a phytase (with reference to the position numbering of SEQ ID NO: 1):-E121T, P394N, D386N, K202N, N204T, Q151N, P153S, P373T, and Q76N.

## Production methods

In further embodiments, the present description also relates to increasing the glycosylation of an enzyme through production methods. In some embodiments the production method used is expression in a filamentous fungi host, for example expression in *Trichoderma* species fungi and specifically for example in *T. reesei.*

The present description also relates to expression of an enzyme in an altered host, wherein alteration has deleted the function of one of more genes, and in some embodiments has deleted the function of the endogenous endo glucosaminidase gene.

In one particular embodiment the present description relates to the expression and production of an enzyme, for example a phytase, and in some embodiments phytase BP17, which may or may not be modified, in *Trichoderma* species fungi, for example in *T. reesei,* wherein the endogenous endo glucosaminidase gene has been deleted.

In some embodiments, the present description relates to the production and/or use of enzymes, such as phytase, with increased glycosylation. In further embodiments increased glycosylation is encouraged by the use of a host with a deleted endoT gene. In some embodiments the enzyme produced has increased stability compared to an enzyme produced using different methods, especially methods using a different host species and/or a host wherein the endogenous endo glucosaminidase gene is not deleted.

Endogenous endo glucosaminidase is a secreted enzyme which removes N-linked glycosylation from other proteins such as phytases. The extent of glycosylation of a protein, such as an enzyme, is greater if it is produced in an endoT-deleted host. According to the description the endoT gene may be deleted by removal, disruption, interference or any method which prevents or reduced the expression and production of EndoT in the host. Exemplary strains are taught for example in WO 09/114380, see for instance Example 5.

In some embodiments the present description relates to the expression and production of a phytase in an endoT deleted host. In further embodiments the phytase is BP17 or a homologue, variant or derivative thereof, for example having more than 75%, for example more than 80%, for example more than 90%, and for further example more than 95%, 96%, 97%, 98%, or 99% identity to BP17 (SEQ ID NO: 1). In some embodiments the host is a filamentous fungi host, for example a *Trichoderma* species fungi and for further example *T. reesei.* In some embodiments the phytase has increased stability, for example thermostability is increased compared to a phytase produced by other production methods, or a phytase which is not glycosylated or not highly glycosylated. The extent of glycosylation can be determined by mass spectrometry.

In some embodiments, the production methods of the present description provide a more thermostable phytase, which therefore increases the post-processing levels of phytase activity present in feed. In some embodiments the feed processing involves the formation of pellets. In further embodiments the pellets comprising the phytase of the current description have increased post-processing levels of phytase activity. In yet further embodiments the phytase of the current description is better able to survive the pelleting process and retain post-processing levels of phytase activity.

## Combinations of embodiments

In a further embodiment, facets of the current description can be combined. In other words, an enzyme of the description which has been modified to increase the number of glycosylation sites by altering the amino acid sequence, may be produced in an altered host, for example a host wherein the endoT gene has been deleted. This maximizes the extent of glycosylation of the enzyme. In some embodiments the host is a *Trichoderma* species fungi, for example *T. reesei.*

In some embodiments, the enzyme expressed in the altered host is a phytase, for example BP17 (SEQ ID NO: 1), or Ronozyme P-(CT), or a homologue, variant or derivative thereof, for example having more than 75%, for example more than 80%, for example more than 90%, and for further example more than 95%, 96%, 97%, 98%, or 99% identity to BP17 (SEQ ID NO: 1), or the commercially available Ronozyme P-(CT), respectively.

In some embodiments, the phytase, however produced or with whatever sequence, is in functional proximity to phytic acid, wherein the phytase and phytic acid are in any of the compositions described herein.

## EXAMPLES

The following examples are intended to illustrate some embodiments of the present teachings. The phytase used in Example 1 and Example 2 is the phytase BP17 having the amino acid sequence as shown in SEQ ID NO: 1, made in the EDT-Trichoderma reesei host. The phytase used in Example 3 is the commercially available phytase Ronozyme P-(CT) from DSM. Calculation of percent recovered activity, phytase activity, and DSC were performed as described in the definitions.

**Example** 1-Fluid Bed Granulation and Pelleting Trials

Phytic acid (Sigma) was incorporated into multi-layered granules comprising phytase to assess whether protection to the phytase against subsequent steam treatment could be achieved. These phytase-containing granules were then mixed with animal feed and passed through an animal feed pelleter that incorporated a steam treatment.

*Fluid Bed Granulation*

Enzyme granules can be produced using a fluid-bed spray process described in US Pat. No. 5324649. Here, a Vector FL-1 fluidized bed spray-coater was used. Phytase was added at 12,000 Units/gram (based on total dry granule weight). Phytic acid was added at 0.10% to 0.40% by weight of the total granule or 15 to 60 mM concentration in the enzyme layer. Sodium sulfate seeds (also referred to as the core) were charged into a fluid bed chamber. The "spray 1" solution was prepared by dissolving or dispersing sucrose, corn starch, phytic acid and either paraffin oil or rapeseed oil or antifoam Foamblast 882® into ultrafiltered phytase concentrate (UFC). Additional water was added to this mixture in order to keep the total concentration of solids about 40%. Constant stirring was required in order to keep the corn starch and paraffin oil or rapeseed oil or antifoam well dispersed in the enzyme UFC.

Spray 1 was applied to the sodium sulfate core already charged to the fluid bed granulator using a top spray process. Spray 1 was applied with an initial spray rate of 6.3 g/min ramping up to a final spray rate of 11 g/min over 30 minutes. The atomization air pressure was held between 35 and 40 psi and the bed temperature was held between 43 and 45°C.

Subsequent sprays (Spray 2, Spray 3, etc.) were prepared by dissolving and/or dispersing all of the components of this spray solution into water so as to form a solution containing approximately 18 to 40% solids. Sprays containing water insoluble oil, antifoam or corn starch required continuous stirring in order to keep these materials well dispersed. Spray 2 was applied with a constant spray rate of 23.9 g/min. The atomization air pressure was held between 34 and 36 psi and the bed temperature was held between 45 and 47°C. Spray 3 was applied with an initial spray rate of 7.2 g/min ramping up to a final spray rate of 9.2 g/min over 15 minutes. The atomization air pressure was 40 psi and the bed temperature was held between 49C and 50C.

Multi-layered granules were harvested after the final spray and phytase activity was determined using above-mentioned the phytase assay.

*Pelleting*

The multi-layered granules were then combined with a mixture of 60% corn meal and 40% soy meal (i.e. the mash) at a ratio of 60 grams of granules to 120 kilograms of corn-soy meal such that the final activity of phytase in the mixture before pelleting was approximately 5 units/gram.

This mixture was then pelleted in an animal feed pelleter. The granules and the corn-soy meal were blended in a horizontal ribbon mixer, for approximately 15 minutes. The pellet mill was a Simon Heesen, mono roll type, fitted with a 17.3 cm inner diameter die, with a pellet hole diameter of 3 mm. Die speed was 500 rpm and was driven by a 7.5 kW motor. The typical feed rate was 300 kg per hour. The temperature in the conditioner was kept at +/-0.1 degrees Celsius, measured at the feed outlet from the conditioner. The conditioner had a cascade type mixer system. Two conditioning temperatures were used: 90C and 95C. Steam inlet pressure was 2 atm, and the temperature in the conditioner was controlled by manual adjustment of three valves that regulate the steam delivery. The residence time in the conditioner was approximately 30 seconds. When the target temperature was reached, the system was run for approximately 5 to 10 minutes before sampling took place. Samples were taken for 1-1.5 minute periods, corresponding to 5-7.5 kg of pelleted feed, and were immediately placed in a cooling box with a perforated bottom and air flow of 1500 cubic meters per hour. After cooling for 15 minutes, the samples were downsized twice using a sample divider, and 1 kg was taken for lab tests.

After pelleting and cooling, the pellets were then ground up and assayed for phytase activity. The unpelleted mixture of corn-soy meal and phytase granule is called the "mash" control and was also assayed for phytase activity. The ratio of recovered activity in the enzyme granule pelleted at either 90C or 95C to the mash control activity is called the percent recovered activity, as described in the definitions.

**Stoichiometry**

In the calculations below in Table 1.1, it can be seen that for a granule loaded with 0.1% w/w phytic acid, the level of phytic acid in the enzyme layer is 10.19 millimoles per kilogram of enzyme layer solids or 10.19 millimolal. Improvements in pelleting performance have been observed as shown in Table 3 when phytic acid has been used in granules from 0.1% w/w to 1.0% w/w (10.19 - 101.9 millimolal).

Thus, at 0.10% w/w phytic acid the concentration of phytic acid in the enzyme layer is 10.19 mM, at 0.20% w/w phytic acid the concentration of phytic acid in the enzyme layer is 20.38 mM, at 0.4% w/w phytic acid the concentration of phytic acid in the enzyme layer is 40.76 mM, at 0.19% w/w the concentration of phytic acid in the enzyme layer is 19.361 mM, at 0.37% w/w the concentration of phytic acid in the enzyme layer is 37.703 mM, at 1% w/w the concentration of phytic acid in the enzyme layer is 101.9 mM, Phytic acid was obtained from Sigma, and originally was greater than 70% IP6 as determined by HPLC, with the balance being IP5, IP4, IP3, IP2, and IP1. (In this Table 1.1 L10263 is L-10-263 which is described below in Table 1.2; SP1 refers to spray 1; SP2 refers to spray 2; SP3 and refers to spray 3. Each "spray" is an aqueous solution or suspension containing the specified component solids, which is sprayed onto the cores within the fluid bed coater).

**Table 1.1**

|  | **Granule Recipe** | **L10263** | 6.7 Kg Basis |  |  |  |  |
|---|---|---|---|---|---|---|---|
|  | **Component** | **Component Payload** | **Mass (g)** | **Phytic Acid MW** |  |  |  |
| **SP1** | Enzyme Solids | 4.62% | 310.69 | 660.04 | Daltons |  |  |
|  | Sucrose | 3.00% | 201.75 |  |  |  |  |
|  | Starch | 6.50% | 437.12 | **Whole Granule Mass Basis** |  |  |  |
|  | Phytic Acid | 0.10% | 6.72 | 6724.95 | grams |  |  |
|  | Paraffin oil | 0.75% | 50.44 |  |  |  |  |
|  |  |  |  | **Mass of Enzyme Layer (less phytic acid)** |  |  |  |
| **SP2** | Na2SO4 | 40.00% | 2689.98 | **1000.00** | **grams** |  |  |
|  |  |  |  |  |  |  |  |
| **SP3** | Talc | 6.00% | 403.50 | **Millimoles of Phytic Acid** |  |  |  |
|  | PVA | 3.00% | 201.75 | 10.19 | mM |  |  |
|  |  |  |  |  |  |  |  |
|  |  |  |  |  |  |  |  |
|  |  |  |  |  |  |  |  |
|  |  |  |  |  |  |  |  |
| **Core** | Na2SO4 | 36.03% | 2423.00 |  |  |  |  |
|  |  |  | 6724.95 |  |  |  |  |

**Granule Formulations**

Table 1.2 summarizes the granule formulations made and tested in pelleting trials.

**Table 1.2**

| Granule Recipe Component | V-10-153 | L-10-263 | V-10-264 | L-10-265 |
|---|---|---|---|---|
| **Spray 1** | | | | |
| Enzyme Solids | 4.78% | 4.62% | 4.62% | 4.62% |
| Sucrose | 6.50% | 3.00% | 3.00% | 3.00% |
| Starch | 6.50% | 6.50% | 6.50% | 6.50% |
| Phytic Acid | 0.00% | 0.10% | 0.20% | 0.40% |
| Paraffin Oil | 1.00% | 0.75% | 0.75% | 0.75% |
| | | | | |
| **Spray 2** | | | | |
| Na2SO4 | 40.00% | 40.00% | 40.00% | 40.00% |
| | | | | |
| **Spray 3** | | | | |
| Talc | 6.00% | 6.00% | 6.00% | 6.00% |
| PVA | 3.00% | 3.00% | 3.00% | 3.00% |
| | | | | |
| **Core** | | | | |
| Na2SO4 | 32.22% | 36.03% | 35.93% | 35.73% |
| | | | | |

| Granule Recipe Component | L-10-231 | V-10-231 |
|---|---|---|
| **Spray 1** | | |
| Enzyme Solids | 3.74% | 3.74% |
| Sucrose | 3.00% | 3.00% |
| Starch | 6.50% | 6.50% |
| Phytic Acid | 0.19% | 0.37% |
| Anti-foam | 1.00% | 1.00% |
| | | |
| **Spray 2** | | |
| Na2SO4 | 40.00% | 40.00% |
| | | |
| **Spray 3** | | |
| Talc | 6.00% | 6.00% |
| PVA | 3.00% | 3.00% |
| | | |
| **Core** | | |
| Na2SO4 | 36.57% | 36.39% |

| Granule Recipe Component | V-11-144 | L-11-095 |
|---|---|---|
| **Spray 1** | | |
| Enzyme Solids | 4.44% | 4.44% |
| Sucrose | 3.00% | 3.00% |
| Starch | 6.50% | 6.50% |
| Phytic Acid | 0.00% | 1.00% |
| Rapeseed Oil | 0.75% | 0.75% |
| | | |
| **Spray 2** | | |
| Na2SO4 | 40.00% | 40.00% |
| | | |
| **Spray 3** | | |
| Talc | 6.00% | 6.00% |
| PVA | 3.00% | 3.00% |
| | | |
| **Core** | | |
| Na2SO4 | 36.31% | 35.31% |

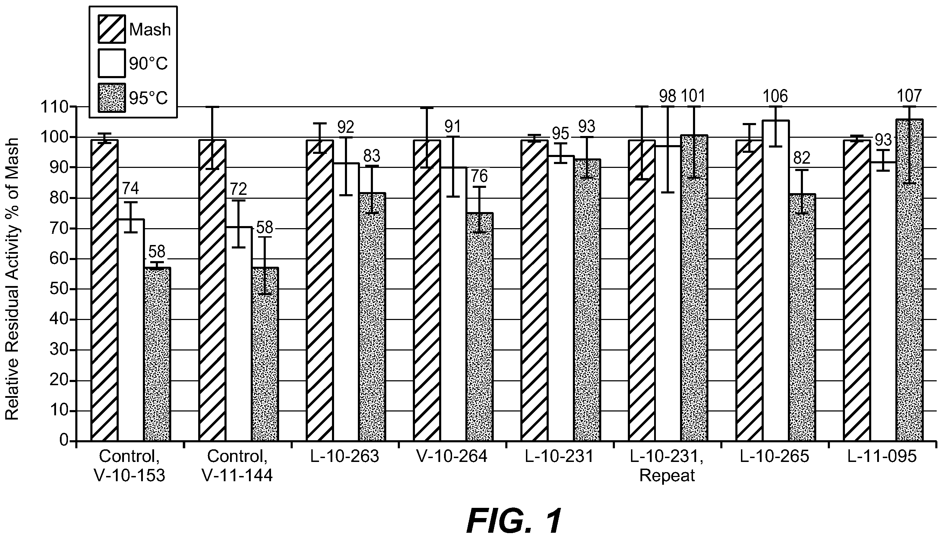

Figure 1 shows the recovered activity obtained after pelleting these Table 1.2 formulations. Pelleting was performed at both 90C and 95C. It can be seen from Figure 1 that when 0.1 to 0.4% Phytic Acid is present in the enzyme layer of the granule, an improvement of recovered activity of between 19 and 27% is observed at 90°C and an improvement of recovered activity of between 17 and 42% is observed at 95°C when compared to the control granule made without any phytic acid in the enzyme layer, i.e. the control phytase. V-10-153 and V-11-144 are the control samples containing no phytic acid. All other samples (L-10-263, V-10-264, L-10-231, L-10-265 and L-11-095) contain between 0.10% and 0.40% phytic acid and show an improvement of recovered activity of between 19 and 27% at 90C and an improvement of recovered activity of between 17 and 42% at 95C. (The variability in improvement does not appear to correlate with the level of phytic acid used in these samples, potentially due to the presence of likely excess at the 0.10% level).

Table 1.3 shows the effect of incubation time of the phytic acid with the phytase UFC prior to spraying onto the granule on the phytase activity post-pelleting at both 90C and 95C. Note that formulations L-11-087, V-11-090, V-11-088 and L-11-088 all have the same composition, which is identical to L-11-095 shown in Table 1.2. The only difference between these formulations was the time they were allowed to incubate prior to spraying onto the granule. It can be seen that for incubation times between 30 minutes and 6 hours there is no change in phytase activity post-pelleting. After 24 hours of incubation a significant decrease in phytase activity post-pelleting is observed at both 90C and 95C, but this time frame is unusually long and would generally not be encountered when making these enzyme granules in a commercial setting.

**Table 1.3 - Phytase Activity Post-Pelleting at 90C and 95C with Varying Incubation** Times

| Formulation | Incubation Time (hrs) | 90°C Recovered Activity | Standard Deviation | 95°C Recovered Activity | Standard Deviation |
|---|---|---|---|---|---|
| L-11-087 | 0.5 | 116% | 18% | 95% | 1% |
| V-11-090 | 3 | 96% | 14% | 63% | 1% |
| V-11-088 | 6 | 95% | 5% | 95% | 1% |
| L-11-088 | 24 | 87% | 1% | 51% | 5% |

**Example 2-Differential Scanning Calorimetry of BP17 Phytase**

DSC was performed on a MicroCal VP-DSC using a scan rate of 90C per hour with a concentration of BP17 phytase of 0.5 mg/ml (0.01mM) in pH 5.5 0.25 M sodium acetate buffer with 20 mM calcium chloride. All phytic acid samples were pH adjusted to 5.5 with sodium hydroxide prior to addition to the acetate buffer and phytase to ensure that all samples were at the same pH of 5.5. The acetate buffer was run in the reference cell of the DSC for all samples.

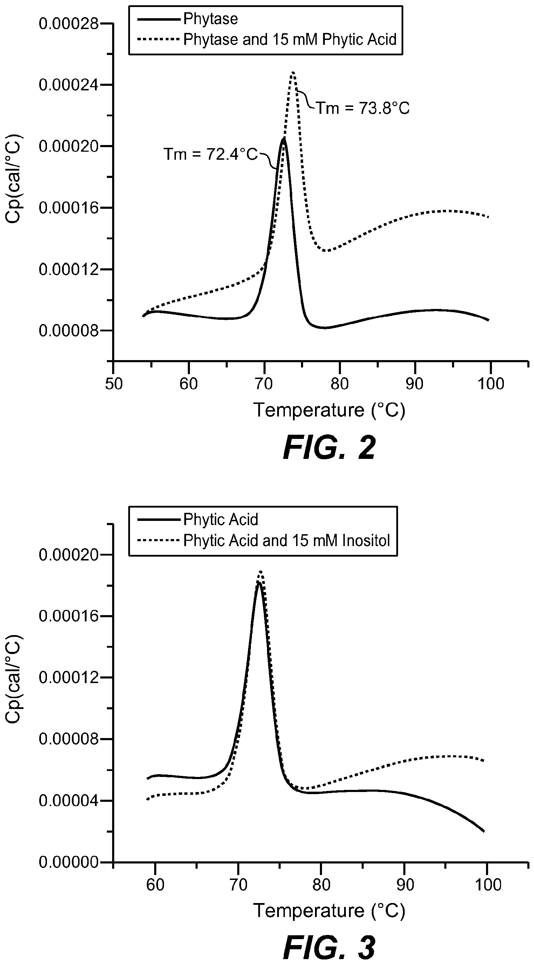

Figure 2 shows the DSC plot for 0.5 mg/ml (0.01mM) BP17 phytase with and without 15 mM phytic acid substrate added. The melting point (Tm) is indicated for each sample and it can be seen that the addition of 15 mM phytic acid increases the Tm from 72.4C to 73.8C (an increase of 1.4C) indicating a stabilization of the native state of phytase.

Figure 3 shows the DSC plot for 15 mM inositol (the fully hydrolyzed product of phytic acid reacting with phytase) and phytase, compared to phytase alone without inositol. It can be seen that inositol does not increase the Tm of phytase. This supports a hypothesis in which the stabilizing entity is not the fully hydrolyzed product of phytase hydrolysis of phytic acid, but instead the partially hydrolyzed phytic acid such as inositol hexaphosphate, inositol pentaphosphate, inositol tetraphosphate, inositol triphosphate, inositol diphosphate, and inositol monophosphate. (The slight height variation of the curves probably relates to the total amount of enzyme undergoing a melting transition. This can vary due to experimental error during sample preparation.)

Granule samples made according to Table 1.2 were analyzed for the distribution of inositol phosphate residues (IP6, IP5, IP4, etc.) using an HPLC based analytical method. The HPLC method is based on ion-exchange using a CarboPac 100 column on a Dionex HPLC-system. A gradient of water and 1 N HCl is run and the eluting material is post-columnn derivitized with a color reagent, 0.1% Fe(NO3)3, 9H2O, forming InsP-Fe complexes detectable with UV at 290nm. This method is only able to quantitate from IP6 down to IP2 with IP1 and IP0 eluting together with the solvent peak. As shown in the below Table 2.1, it was not possible to detect any phytate (IP6) in either of the samples. For the 0.1% phytic acid and 0.5 mg/ml phytase granule samples a small amount of IP3 was present. The liquid samples did not give rise to any peaks apart from the "solvent front". This peak was quite big for the most concentrated of the samples, which suggests total degradation to IP1, IP0 and inorganic phosphate.

Table 2.1 shows that in the granule phytic acid has hydrolyzed to IP3, IP2, IP1 and IP0, and, that no IP6 is present. This supports the hypothesis that the stabilizing entity is IP3, IP2, IP1 (IP0 was eliminated based on DSC data; IP0 is unlikely to be the stabilizing entity because when IP0 (inositol) was tested with phytase using DSC as is shown in Figure 3, no increase in Tm was observed).

The enzyme layer contains 12,000 U/g phytase. Units in the table are HPLC peak areas. A dash (-) indicates that the value was not determined.

| Sample | IP6 | IP5 | IP4 | IP3 | IP2 | IP1 | IP0 |
|---|---|---|---|---|---|---|---|
| L-11-087 | 0 | 0 | 0 | 0.0241 | 0.0004 | - | - |
| L-11-087 | 0 | 0 | 0 | 0.0315 | 0 | - | - |
| L-11-087 | 0 | 0 | 0 | 0.0359 | 0 | - | - |
| L-11-087 | 0 | 0 | 0 | 0.0343 | 0 | - | - |
| L-11-088 | 0 | 0 | 0 | 0.0288 | 0 | - | - |
| L-11-088 | 0 | 0 | 0 | 0.0209 | 0 | - | - |
| 100 mg/ml phytic acid and 0.5 mg/ml phytase | 0 | 0 | 0 | 0 | 0 | - | - |
| 100 mg/ml phytic acid and 0.5 mg/ml phytase | 0 | 0 | 0 | 0 | 0 | - | - |

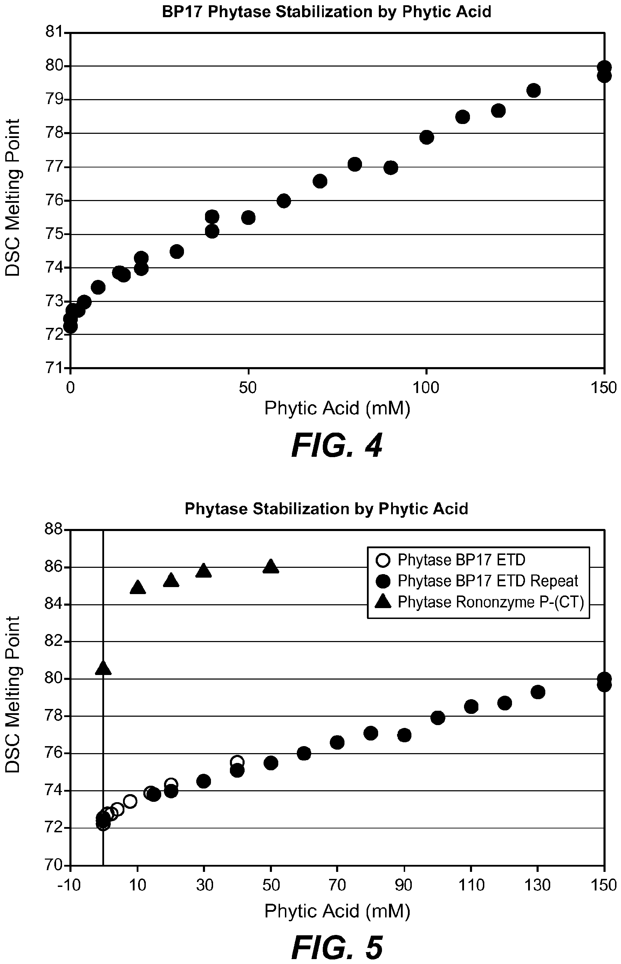

Figure 4 shows a plot of the DSC Tm determined for phytase in the presence of phytic acid at a concentration between 0 and 150 mM. It can be seen that the Tm of phytase increases from 72.4C to 80C at 150 mM phytic acid, showing a 7.6C increase in Tm over phytase without phytic acid added. This is indicative of a very strong stabilizing effect from the phytic acid. It was not possible to determine the Tm of phytase when phytic acid was higher than 150 mM because at this concentration the phytic acid was interfering with the DSC measurement of the phytase.

**Example 3-Differential Scanning Calorimetry of Ronozyme P-CT**

To explore the generalizability of the phytic acid stabilization on other phyases, a DSC experiment was performed using phytase extracted from DSM's granular Rononzyme P-(CT) product.

The phytase enzyme was extracted from the granule by dissolving it in pH 5.5 sodium acetate buffer at a concentration of 10 grams granule solids per 100 grams of buffer for 1 hour. This material was then filtered through a 0.20 micron filter and then buffer exchanged 5 times using pH 5.5 sodium acetate buffer and a 10 K molecular weight cutoff Centricon®tube. Total protein concentration was measured using a Lowry type assay on a Konelab® automated chemical analyzer.

In Figure 5, one sees a similar type of stabilization (Tm increase) in the presence of phytic acid as was earlier observed with BP17.

The higher Tm is likely because this phytase is a different enzyme sequence than the BP17 of previous examples. The effect of phytic acid on Tm is similar to BP17 phytase and the slopes appear similar.

## SEQUENCES

SEQ ID NO: 1

NDTPASGYQVEKVVILSRHGVRAPTKMTQTMRDVTPNTWPEWPVKLGYITPRGEHLISLMGG
FYRQKFQQQGILSQGSCPTPNSI
YVWTDVAQRTLKTGEAFLAGLAPQCGLTIHHQQNLEKADPLFHPVKAGICSMDKTQVQQAVE
KEAQTPIDNLNQHYIPSLALMNT
TLNFSKSPWCQKHSADKSCDLGLSMPSKLSIKDNGNEVSLDGAIGLSSTLAEIFLLEYAQGMP
QAAWGNIHSEQEWALLLKLHNV
YFDLMERTPYIARHKGTPLLQAISNALNPNATESKLPDISPDNKILFIAGHDTNIANIAGMLNMRW
TLPGQPDNTPPGGALVFER
LADKSGKQYVSVSMVYQTLEQLRSQTPLSLNQPAGSVQLKIPGCNDQTAEGYCPLSTFTRVV
SQSVEPGCQLQ

SEQ ID NO: 2

NDTPASGYQVEKVVILSRHGVRAPTKMTQTMRDVTPNTWPEWPVKLGYITPRGEHLISLMGG
FYRQKFQQQGILSQGSCPTPNSI
YVWTDVDQRTLKTGEAFLAGLAPQCGLTIHHQQNLEKADPLFHPVKAGICSMDKTQVQQAVE
KEAQTPIDNLNQHYIPSLALMNT
TLNFSKSPWCQKHSADKSCDLGLSMPSKLSIKDNGNEVSLDGAIGLSSTLAEIFLLEYAQGMP
QAAWGNIHSEQEWALLLKLHNV
YFDLMERTPYIARHKGTPLLQAISNALNPNATESKLPDISPDNKILFIAGHDTNIANIAGMLNMRW
TLPGQPDNTPPGGALVFER
LADKSGKQYVSVSMVYQTLEQLRSQTPLSLNQPAGSVQLKIPGCNDQTAEGYCPLSTFTRVV
SQSVEPGCQLQ

SEQ ID NO: 3

NDTPASGYQVEKVVILSRHGVRAPTKMTQTMRDVTPYTWPEWPVKLGYITPRGEHLISLMGG
FYRQKFQQQGILPRGSCPTPNSI
YVWTDVAQRTLKTGEAFLAGLAPQCGLTIHHQQNLEKADPLFHPVKAGICSMDKTQVQQAVE
KEAQTPIDNLNQRYIPELALMNT
ILNFSKSPWCQKHSADKPCDLALSMPSKLSIKDNGNEVSLDGAIGLSSTLAEIFLLEYAQGMPQ
VAWGNIHSEQEWALLLKLHNV
YFDLMERTPYIARHKGTPLLQAISNALNPNATESKLPDISPDNKILFIAGHDTNIANIAGMLNMRW
TLPGQPDNTPPGGALVFER
LADKSGKQYVSVSMVYQTLEQLRSQTPLSLNQPPGSVQLKIPGCNDQTAEGYCPLSTFTRVV
SQSVEPGCQLQ

SEQ ID NO: 4

NDTPASGYQVEKVVILSRHGVRAPTKMTQTMRDVTPNTWPEWPVKLGYITPRGEHLISLMGG
FYRQKFQQQGILSQGSCPTPNSI
YVWWADVDQRTLKTGEAFLAGLAPQCGLTIHHQQNLEKADPLFHPVKAGTCSMDKTQVQQAVE
KEAQTPIDNLNQHYIPFLALMNT
TLNFSTSAWCQKHSADKSCDLGLSMPSKLSIKDNGNKVALDGAIGLSSTLAEIFLLEYAQGMPQ
AAWGNIHSEQEWASLLKLHNV
QFDLMARTPYIARHNGTPLLQAISNALNPNATESKLPDISPDNKILFIAGHDTNIANIAGMLNMR
WTLPGQPDNTPPGGALVFER
LADKSGKQYVSVSMVYQTLEQLRSQTPLSLNQPAGSVQLKIPGCNDQTAEGYCPLSTFTRVV
SQSVEPGCQLQ

SEQ ID NO: 5 (E121T)

NDTPASGYQVEKVVILSRHGVRAPTKMTQTMRDVTPNTWPEWPVKLGYITPRGEHLISLMGG
FYRQKFQQQGILSQGSCPTPNSIYVWTDVAQRTLKTGEAFLAGLAPQCGLTIHHQQNL**T**KADP
LFHPVKAGICSMDKTQVQQAVEKEAQTPIDNLNQHYIPSLALMNTTLNFSKSPWCQKHSADKS
CDLGLSMPSKLSIKDNGNEVSLDGAIGLSSTLAEIFLLEYAQGMPQAAWGNIHSEQEWALLLKL
HNVYFDLMERTPYIARHKGTPLLQAISNALNPNATESKLPDISPDNKILFIAGHDTNIANIAGMLN
MRWTLPGQPDNTPPGGALVFERLADKSGKQYVSVSMVYQTLEQLRSQTPLSLNQPAGSVQL
KIPGCNDQTAEGYCPLSTFTRVVSQSVEPGCQLQ

SEQ ID NO: 6 (P394N)

NDTPASGYQVEKVVILSRHGVRAPTKMTQTMRDVTPNTWPEWPVKLGYITPRGEHLISLMGG
FYRQKFQQQGILSQGSCPTPNSIYVWTDVAQRTLKTGEAFLAGLAPQCGLTIHHQQNLEKADP
LFHPVKAGICSMDKTQVQQAVEKEAQTPIDNLNQHYIPSLALMNTTLNFSKSPWCQKHSADKS
CDLGLSMPSKLSIKDNGNEVSLDGAIGLSSTLAEIFLLEYAQGMPQAAWGNIHSEQEWALLLKL
HNVYFDLMERTPYIARHKGTPLLQAISNALNPNATESKLPDISPDNKILFIAGHDTNIANIAGMLN
MRWTLPGQPDNTPPGGALVFERLADKSGKQYVSVSMVYQTLEQLRSQTPLSLNQPAGSVQL
KIPGCNDQTAEGYC**N**LSTFTRVVSQSVEPGCQLQ

SEQ ID NO: 7 (D386N)

NDTPASGYQVEKVVILSRHGVRAPTKMTQTMRDVTPNTWPEWPVKLGYITPRGEHLISLMGG
FYRQKFQQQGILSQGSCPTPNSIYVWTDVAQRTLKTGEAFLAGLAPQCGLTIHHQQNLEKADP
LFHPVKAGICSMDKTQVQQAVEKEAQTPIDNLNQHYIPSLALMNTTLNFSKSPWCQKHSADKS
CDLGLSMPSKLSIKDNGNEVSLDGAIGLSSTLAEIFLLEYAQGMPQAAWGNIHSEQEWALLLKL
HNVYFDLMERTPYIARHKGTPLLQAISNALNPNATESKLPDISPDNKILFIAGHDTNIANIAGMLN
MRWTLPGQPDNTPPGGALVFERLADKSGKQYVSVSMVYQTLEQLRSQTPLSLNQPAGSVQL
KIPGCN**N**QTAEGYCPLSTFTRVVSQSVEPGCQLQ

SEQ ID NO: 8 K202N and N204T)

NDTPASGYQVEKVVILSRHGVRAPTKMTQTMRDVTPNTWPEWPVKLGYITPRGEHLISLMGG
FYRQKFQQQGILSQGSCPTPNSIYVWTDVAQRTLKTGEAFLAGLAPQCGLTIHHQQNLEKADP
LFHPVKAGICSMDKTQVQQAVEKEAQTPIDNLNQHYIPSLALMNTTLNFSKSPWCQKHSADKS
CDLGLSMPSKLSI**ND**T**GNEVSLDGAIGLSSTLAEIFLLEYAQGMPQAAWGNIHSEQEWALLLKL
HNVYFDLMERTPYIARHKGTPLLQAISNALNPNATESKLPDISPDNKILFIAGHDTNIANIAGMLN
MRWTLPGQPDNTPPGGALVFERLADKSGKQYVSVSMVYQTLEQLRSQTPLSLNQPAGSVQL
KIPGCNDQTAEGYCPLSTFTRVVSQSVEPGCQLQ

SEQ ID NO: 9 (Q151N and P153S)

NDTPASGYQVEKVVILSRHGVRAPTKMTQTMRDVTPNTWPEWPVKLGYITPRGEHLISLMGG
FYRQKFQQQGILSQGSCPTPNSIYVWTDVAQRTLKTGEAFLAGLAPQCGLTIHHQQNLEKADP
LFHPVKAGICSMDKTQVQQAVEKEA**NTS**IDNLNQHYIPSLALMNTTLNFSKSPWCQKHSADKS
CDLGLSMPSKLSIKDNGNEVSLDGAIGLSSTLAEIFLLEYAQGMPQAAWGNIHSEQEWALLLKL
HNVYFDLMERTPYIARHKGTPLLQAISNALNPNATESKLPDISPDNKILFIAGHDTNIANIAGMLN
MRWTLPGQPDNTPPGGALVFERLADKSGKQYVSVSMVYQTLEQLRSQTPLSLNQPAGSVQL
KIPGCNDQTAEGYCPLSTFTRVVSQSVEPGCQLQ

SEQ ID NO: 10 (P373T)

NDTPASGYQVEKVVILSRHGVRAPTKMTQTMRDVTPNTWPEWPVKLGYITPRGEHLISLMGG
FYRQKFQQQGILSQGSCPTPNSIYVWTDVAQRTLKTGEAFLAGLAPQCGLTIHHQQNLEKADP
LFHPVKAGICSMDKTQVQQAVEKEAQTPIDNLNQHYIPSLALMNTTLNFSKSPWCQKHSADKS
CDLGLSMPSKLSIKDNGNEVSLDGAIGLSSTLAEIFLLEYAQGMPQAAWGNIHSEQEWALLLKL
HNVYFDLMERTPYIARHKGTPLLQAISNALNPNATESKLPDISPDNKILFIAGHDTNIANIAGMLN
MRWTLPGQPDNTPPGGALVFERLADKSGKQYVSVSMVYQTLEQLRSQTPLSLNQ**T**AGSVQL
KIPGCNDQTAEGYCPLSTFTRVVSQSVEPGCQLQ

SEQ ID NO: 11 (Q76N)

NDTPASGYQVEKVVILSRHGVRAPTKMTQTMRDVTPNTWPEWPVKLGYITPRGEHLISLMGG
FYRQKFQQQGILS**N**GSCPTPNSIYVWTDVAQRTLKTGEAFLAGLAPQCGLTIHHQQNLEKADP
LFHPVKAGICSMDKTQVQQAVEKEAQTPIDNLNQHYIPSLALMNTTLNFSKSPWCQKHSADKS
CDLGLSMPSKLSIKDNGNEVSLDGAIGLSSTLAEIFLLEYAQGMPQAAWGNIHSEQEWALLLKL
HNVYFDLMERTPYIARHKGTPLLQAISNALNPNATESKLPDISPDNKILFIAGHDTNIANIAGMLN
MRWTLPGQPDNTPPGGALVFERLADKSGKQYVSVSMVYQTLEQLRSQTPLSLNQPAGSVQL
KIPGCNDQTAEGYCPLSTFTRVVSQSVEPGCQLQ

SEQ ID NO: 12
MTISAFNRKKLTLHPGLFVALSAIFSLGSTAYA

## Claims

1. A granule composition comprising phytic acid and phytase, wherein the phytase and the phytic acid are in adjacent coating layers of a multi-layered granule, or phytase is in the core of the granule and phytic acid is in the adjacent coating layer, wherein the phytic acid is at least 10 millimolal in said composition, and wherein the phytic acid is selected from inositol hexaphosphate, inositol pentaphosphate, inositol tetraphosphate, inositol triphosphate, inositol diphosphate and inositol monophosphate, or salts or mixtures thereof.

2. The composition according to claim 1 wherein the phytic acid is at least 20 millimolal, at least 30 millimolal, at least 50 millimolal, at least 80 millimolal, or at least 100 millimolal, wherein the phytase is present at no more than 500 millimolal, 400 millimolal, 300 millimolal, 200 millimolal, 150 millimolal, or 100 millimolal.

3. The composition according to claim 2 wherein the phytic acid is 10-100 millimolal, 20-90 millimolal, 30-80 millimolal, or 40-70 millimolal.

4. The composition according to any of claims 1 to 3, wherein the recovered activity of the phytase is at least 15%, at least 17%, at least 19%, at least 20%, at least 23%, at least 25%, at least 27%,or at least 30% higher, after admixing the granule with at least one food or animal feed ingredient and pelleting the resulting admixture with steam treatment, as compared to a control phytase not stabilized by phytic acid.

5. The composition according to any of claims 1 to 4, wherein the phytase is phytase BP17.

6. The composition according to any of claims 1 to 5, wherein the phytase is phytase BP17 produced in a *Trichoderma* host cell that comprises a deletion of the endo-N-acetyl glucosaminidase gene.

7. A pellet composition comprising a granule composition according to any one of claims 1 to 6.

8. A method for producing a heat-treated food or animal feed pellet comprising the steps of:

admixing a granule composition according to any one of claims 1 to 5 with at least one food or animal feed ingredient; and
pelleting the resulting admixture with steam.

**Patentansprüche**

1. Granulare Zusammensetzung, umfassend Phytinsäure und Phytase, wobei sich die Phytase und die Phytinsäure in benachbarten Beschichtungsschichten eines mehrschichtigen Granulums befinden, oder Phytase sich im Kern des Granulums befindet und Phytinsäure sich in der benachbarten Beschichtungsschicht befindet, wobei die Phytinsäure in der Zusammensetzung mindestens 10 millimolal ist, und wobei die Phytinsäure ausgewählt ist aus Inositolhexaphosphat, Inositolpentaphosphat, Inositoltetraphosphat, Inositoltriphosphat, Inositoldiphosphat und Inositolmonophosphat oder Salzen oder Mischungen davon.

2. Zusammensetzung nach Anspruch 1, wobei die Phytinsäure mindestens 20 millimolal, mindestens 30 millimolal, mindestens 50 millimolal, mindestens 80 millimolal oder mindestens 100 millimolal ist, wobei die Phytase in nicht mehr als 500 millimolal, 400 millimolal, 300 millimolal, 200 millimolal, 150 millimolal oder 100 millimolal vorhanden ist.

3. Zusammensetzung nach Anspruch 2, wobei die Phytinsäure 10-100 millimolal, 20-90 millimolal, 30-80 millimolal oder 40-70 millimolal ist.

4. Zusammensetzung nach einem der Ansprüche 1 bis 3, wobei die wiedergefundene Aktivität der Phytase mindestens 15 %, mindestens 17 %, mindestens 19 %, mindestens 20 %, mindestens 23 %, mindestens 25 %, mindestens 27 % oder mindestens 30 % höher ist, nachdem das Granulum mit mindestens einem Nahrungsmittel- oder Tierfutterbestandteil gemischt und die resultierende Mischung mit Dampfbehandlung pelletiert wurde, verglichen mit einer Kontrollphytase, die nicht mit Phytinsäure stabilisiert wurde.

5. Zusammensetzung nach einem der Ansprüche 1 bis 4, wobei die Phytase Phytase BP17 ist.

6. Zusammensetzung nach einem der Ansprüche 1 bis 5, wobei die Phytase Phytase BP17 ist, die in einer *Tri*choderma-Wirtszelle produziert wurde, die eine Deletion des endo-N-Acetylglucosaminidase-Gens aufweist.

7. Pelletzusammensetzung, die eine granulare Zusammensetzung gemäß einem der Ansprüche 1 bis 6 umfasst.

8. Verfahren zur Herstellung eines wärmebehandelten Nahrungsmittel- oder Tierfutterpellets, umfassend die Schritte:

Mischen einer granularen Zusammensetzung gemäß einem der Ansprüche 1 bis 5 mit mindestens einem Nahrungsmittel- oder Tierfutterbestandteil; und
Pelletieren der resultierenden Mischung mit Dampf.

**Revendications**

1. Composition de granulé comprenant un acide phytique et une phytase, la phytase et l'acide phytique étant dans des couches de revêtement adjacentes d'un granulé multicouche, ou une phytase étant dans le noyau du granulé et l'acide phytique étant dans la couche de revêtement adjacente, l'acide phytique étant au moins 10 millimolaires dans ladite composition, et l'acide phytique étant choisi parmi l'hexaphosphate d'inositol, le pentaphosphate d'inositol, le tétraphosphate d'inositol, triphosphate d'inositol, le diphosphate d'inositol et le monophosphate d'inositol, ou des sels ou des mélanges correspondants.

2. Composition selon la revendication 1, l'acide phytique étant au moins 20 millimolaires, au moins 30 millimolaires, au moins 50 millimolaires, au moins 80 millimolaires, ou au moins 100 millimolaires, la phytase étant présente à raison de pas plus de 500 millimolaires, 400 millimolaires, 300 millimolaires, 200 millimolaires, 150 millimolaires, ou 100 millimolaires.

3. Composition selon la revendication 2, l'acide phytique étant 10 à 100 millimolaires, 20 à 90 millimolaires, 30 à 80 millimolaires, ou 40 à 70 millimolaires.

4. Composition selon l'une quelconque des revendications 1 à 3, l'activité récupérée de la phytase étant d'au moins 15 %, au moins 17 %, au moins 19 %, au moins 20 %, au moins 23 %, au moins 25 %, au moins 27 %, ou au moins 30 % plus élevée, après mélange du granulé avec au moins un ingrédient de produit alimentaire ou de produit alimentaire pour animaux et mise en pastilles du mélange résultant avec traitement à la vapeur, par comparaison avec une phytase témoin non stabilisée par un acide phytique.

5. Composition selon l'une quelconque des revendications 1 à 4, la phytase étant une phytase BP17.

6. Composition selon l'une quelconque des revendications 1 à 5, la phytase étant une phytase BP17 produite dans une cellule hôte de *Trichoderma* qui comprend une délétion du gène endo-N-acétylglucosaminidase.

7. Composition de pastille comprenant une composition de granulé selon l'une quelconque des revendications 1 à 6.

8. Procédé pour la production d'une pastille de produit alimentaire ou de produit alimentaire pour animaux traitée thermiquement comprenant les étapes de :

mélange d'une composition de granulé selon l'une quelconque des revendications 1 à 5 avec au moins un ingrédient de produit alimentaire ou de produit alimentaire pour animaux ; et
mise en pastilles du mélange résultant avec de la vapeur.

*FIG. 1*

**FIG. 2**

**FIG. 3**

**BP17 Phytase Stabilization by Phytic Acid**

*FIG. 4*

**Phytase Stabilization by Phytic Acid**

Legend:
- ○ Phytase BP17 ETD
- ● Phytase BP17 ETD Repeat
- ▲ Phytase Rononzyme P-(CT)

*FIG. 5*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0969089 A
- EP 0619369 A
- US 5554399 A
- WO 2004071218 A
- WO 03037102 A
- EP 0420428 A
- US 4952396 A
- US 5112814 A
- US 4758430 A
- US 20100004170 A
- WO 9316175 A
- WO 2006043178 A
- US 6110719 A
- WO 09114380 A
- US 4689297 A
- US 5324649 A
- EP 656058 B1
- US 454332 A
- US 6248706 B
- EP 804532 B1
- US 6534466 B
- US 20100124586 A
- WO 9932595 A

### Non-patent literature cited in the description

- **KLIBANOV.** Stabilization of Enzymes against Thermal Inactivation. *Advances in Applied Microbiology,* 1983, vol. 29, 1-28
- **PACE ; GRATH.** *J Biol Chem,* 1980, vol. 255, 3862
- **SINGH ; SATYANARAYANA.** *Bioresource Technology,* 2009, vol. 100, 2046-2051
- **SAMBROOK et al.** Molecular Cloning: A Laboratory Manual. 1989
- Oligonucleotide Synthesis. 1984
- Current Protocols in Molecular Biology. 1994
- PCR: The Polymerase Chain Reaction. 1994
- **KRIEGLER.** Gene Transfer and Expression: A Laboratory Manual. 1990
- Pelleting Cost Center. **FAIRFIELD, D.** Feed Manufacturing Technology IV. American Feed Industry Association, 1994, 110-139
- **SINGLETON et al.** Dictionary of Microbiology and Molecular Biology. John Wiley and Sons, 1994
- **HALE ; MARKHAM.** The Harper Collins Dictionary of Biology. Harper Perennial, 1991
- **ALEXOPOULOS, C. J.** Introductory Mycology. Wiley, 1962
- Pelleting Cost Center. **FAIRFIELD, D.** Feed Manufacturing Technology IV. American Feed Industry Association, 1994, 110-139
- **ENGELEN A J ; VAN DER HEEFT F C ; RANDSDORP P H ; SOMERS W A ; SCHAEFER J ; VAN DER VAT B J.** Determination of phytase activity in feed by a colorimetric enzymatic method: collaborative interlaboratory study. *J AOAC Int.,* May 2001, vol. 84 (3), 629-33